# EUROPÄISCHE PATENTSCHRIFT

(11) **EP 1 100 873 B1**
(45) Veröffentlichungstag und Bekanntmachung des Hinweises auf die Patenterteilung: **01.04.2009**
(21) Anmeldenummer: 99944299.9
(22) Anmeldetag: 27.07.1999
(51) Int. Cl.: C12N 5/06, C12M 3/06, C12Q 1/68, A61K 35/12

(54) **KREBSZELLEN AUS ZELLHALTIGEN KÖRPERFLÜSSIGKEITEN, DEREN ISOLIERUNG, VERWENDUNG SOWIE DIESE ENTHALTENDE MITTEL**
CANCER CELLS FROM BODY FLUIDS CONTAINING CELLS, ISOLATION THEREOF AND AGENTS CONTAINING THE SAME
CELLULES CANCEREUSES PROVENANT DE LIQUIDES DU CORPS CONTENANT DES CELLULES, LEUR ISOLATION, LEUR UTILISATION ET LES PRODUITS LES CONTENANT

(30) Priorität: 27.07.1998 DE 19833738
(43) Veröffentlichungstag der Anmeldung: 23.05.2001
(73) Patentinhaber: Giesing, Michael, 45659 Recklinghausen (DE)
(72) Erfinder: GIESING, Michael, D-45659 Recklinghausen (DE); AUSTRUP, Frank, D-45663 Recklinghausen (DE)
(74) Vertreter: Kinzebach, Werner
(86) Internationale Anmeldenummer: PCT/EP1999/005386
(87) Internationale Veröffentlichungsnummer: WO 2000/006702

(56) Entgegenhaltungen:
- EP-A- 0 483 506
- EP-A- 0 584 715
- EP-A- 0 806 666
- WO-A-99/10528
- HIRTE, H.W. ET AL.: "A rapid and simple method for the purification of tumor cells from ascites fluid of ovarian carcinoma" GYNECOLOGIC ONCOLOGY, Bd. 44, 1992, Seiten 223-226, XP002122420
- SHANTZ, G.D. ET AL.: "Tumour metastsis and cell deformation: Assessment of nucleopore filtration" TREATMENT OF METASTSIS: PROBLEMS AND PROSPECTS,1985, Seiten 291-294, XP002122422
- PITTMAN K ET AL: "Reverse transcriptase-polymerase chain reaction for expression of tyrosinase to identify malignant melanoma cells in peripheral blood" ANNALS OF ONCOLOGY,NL,KLUWER, DORDRECHT, Bd. 7, 1996, Seite 297-301 XP002092615 ISSN: 0923-7534
- RYE, P.D. ET AL.: "Immunobead filtration: A novel approach for the isolation and propagation of tumor cells" THE AMERICAN JOURNAL OF PATHOLOGY, Bd. 150, Nr. 1, 1997, Seiten 99-106, XP002122421

## Beschreibung

Die vorliegende Erfindung betrifft ein Verfahren zur Isolierung von Krebszellen aus zellhaltigen Körperflüssigkeiten; aus Körperflüssigkeiten isolierte Krebszellen umfassende Zellgemische; Verfahren zur Etablierung entsprechender Zellinien oder zur Isolierung entsprechender Zellbestandteile; deren Verwendung als Therapeutika ; und diese enthaltende pharmazeutische oder tierarzneiliche Mittel.

Das erfindungsgemäße Verfahren beruht darauf, daß in einer zellhaltigen Körperflüssigkeit Zellen und Zellaggregate unterschiedlicher Größe und Gestalt anzutreffen sind. Die Isolierung und Charakterisierung von Krebszellen ist vor allem im Bereich der Onkologie für die Beantwortung diagnostischer, prognostischer, therapeutischer und wissenschaftlicher Fragestellungen sowohl im tierexperimentellen als auch im humanmedizinischen Bereich von großer Bedeutung. Darüber hinaus dient das erfindungsgemäße Verfahren zur, gegebenenfalls vollständigen, Entnahme von Krebszellen aus zellhaltigen Flüssigkeiten oder zellhaltigen Fraktionen (Isolaten) davon. Die isolierten Krebszellen, daraus etablierte Zellinien und abgeleitete Zellbestandteile spiegeln einen im wesentlichen nativen, d.h. biologischen Zustand wieder, der entsprechenden Krebszellen in der Körperflüssigkeit zugeordnet werden kann.

Die Isolierung von Krebszellen zur Durchführung von in-vitro oder ex-vivo Untersuchungen ist beispielsweise dann unproblematisch, wenn ein Primärtumor lokalisiert wurde und somit eine Gewebeprobe der Untersuchung zugrundegelegt werden kann. In diesem Sinne beschreibt beispielsweise das US-Patent 5,242,806 einen Chemosensibilitätstest an Tumorzellen aus Biopsiematerial. Auch das in dem US-Patent 5,023,172 beschriebene MTS-System zum Austesten von tumorinaktivierenden Wirkstoffen geht von Biopsiematerial aus. Dieses wird mit Trypsin behandelt und anschließend zu einer Feeder-Zellen-Suspension gegeben, um multizelluläre Tumorsphäroide zu bilden. In diesem Zusammenhang kann auch die methodische Arbeit von Shantz G.D. et al., Treatment of Metastasis: Problems and Prospects, 1985, 291-294, genannt werden. Auch hier werden Melanom-Zellinien, die ausgehend von Zellmaterial eines Primärtumors etabliert wurden, untersucht. Diese Zellen werden über einen Filter mit einem Porendurchmesser von 10 und 12 µm gegeben, wobei die Zeit bestimmt wird, bis im Wesentlichen sämtliche Zellen den Filter passiert haben. Da die für die Filtration benötigte Zeit von der passiven Deformierbarkeit der Melanomzellen abhängen soll, wird versucht, besagte Deformierbarkeit mit dem metastatischen Potenzial der Melanomzellen zu korrelieren.

Ist hingegen kein Tumor lokalisiert worden und steht deshalb kein Gewebe zur Verfügung, so ergeben sich Probleme. Zwar weiß man, daß sich unter Umständen auch in Körperflüssigkeiten Krebszellen aufspüren lassen, aufgrund der geringen Konzentration an Krebszellen in Körperflüssigkeiten sind entsprechende Analysen jedoch technisch extrem schwierig. Deshalb wird die Aussagekraft derartiger Analysen vor allem durch die klassische Medizin häufig in Zweifel gezogen.

So kann im Rahmen der Identifizierung und Charakterisierung disseminierter Krebszellen - wozu insbesondere tumoröse Zellen gehören, die sich vom Primärtumor, von Metastasen und/oder Rezidiven abgelöst haben und in Körperflüssigkeiten zirkulieren - die Isolierung von Krebszellen entscheidende Bedeutung haben. Mißt man beispielsweise die Expression relevanter Gene durch Zellen einer zu untersuchenden Körperflüssigkeit, so ist eine Isolierung von Krebszellen dann nicht erforderlich, wenn die relevanten Gene von den üblicherweise in der Körperflüssigkeiten vorhandenen, nicht entarteten Zellen nicht oder nur in sehr geringem Maße exprimiert werden (vergleiche beispielsweise in Pittman K. et al, Annals of Oncology, 7, 1996, 297-301). Werden dagegen die relevanten Gene auch von den nicht entarteten Zellen exprimiert, ist es erforderlich, disseminerte Krebszellen zunächst zu isolieren und dann die Expression relevanter Gene zu messen. In diesem Falle bietet eine quantitative Analyse zur Expression bestimmter z.B. tumorbiologisch relevanter Nukleinsäuren (z.B. FAS-Ligand, FAS-Rezeptor, bax, bcl-2, Ki-67, Cycline, Adhesionsmoleküle) die Möglichkeit der Expressionszuordnung zum Tumorisolat.

Zwecks Charakterisierung der Tumorzellen ist es sinnvoll, genomische Veränderungen der Tumorzellen auf DNA-Ebene zu analysieren. Bestimmungen wie "LOH (loss of heterocygocity), Mutationen, Amplifikationen etc." bedürfen einer extrem reinen Population von Tumorzellen, da verunreinigende "Wildtyp-Zellen" potentielle Genomveränderungen überdecken und so diese nicht mehr detektierbar machen. Durch das vorliegende Verfahren werden kontaminierende, wildtypexprimierende Zellen wenigstens soweit entfernt, daß genomische Veränderungen der Krebszellen meßbar sind. Wildtyp-Zellen, z.B. CD45-positive Zellen, können dann als Bezugsgröße zur Messung von z.B. LOH's, Amplifikationen und Mutationen dienen.

Bekannte zu Isolationszwecken angebotene Verfahren führen vielfach zu einer lediglich unspezifischen Anreicherung von Krebszellen. Auch das in der US-A-5,529,903 (entspricht EP-A-0 584 715) beschriebene Leukopherese-Verfahren bietet keine spezifische Anreicherung von Krebszellen, sondern liefert Fraktionen, die zum überwiegenden Teil aus mononukleären Zellen bestehen, wie sie auch mit herkömmlichen Dichtegradienten erhalten werden können.

Die DE 40 062 93 A1 gibt ein Trennmittel aus Polyvinylacetalharz an, mit dem Zellen aus Suspensionen abgetrennt werden können. Dieses Verfahren ist eine Weiterentwicklung der schon lange bekannten Nylonwolle-Aufreinigung von T-Lymphozyten. Grundprinzip ist auch hier die präferentielle Adsorption von B-Zellen und Makrophagen/Monozyten an das Trennmittel.

Auch mit dem in der europäischen Patentanmeldung EP 0 448 837 A2 beschriebenen Verfahren werden Zellen aus einer Suspension unspezifisch auf einem Filter aufkonzentriert, wobei der dazu angelegte Druck Rückschlüsse auf die Zellzahl zuläßt.

Zur Vermeidung von gynäkologischen Abstrichen schlägt die EP 0 483 506 A1 vor, Menstruationsblut zu filtrieren. Der Filter wird so gewählt, daß rote und weiße Blutzellen die Filterporen passieren können. Die übrigen, auf dem Filter zurückgehaltenen Zellen werden dann einer Papanicolaou-Färbung unterzogen, und es wird die übliche cytodiagnostische Beurteilung des Zellbildes vorgenommen, um normale von anormalen Zellen zu unterscheiden.

Zum raschen Sammeln und Konzentrieren von Zellen aus großen Flüssigkeitsmengen wie Mundspülungen dient das in dem US-Patent 5,578,459 beschriebene Verfahren. Eine Auftrennung von Zellgemischen wird nicht gelehrt. Auch nach dem in der japanischen Anmeldung JP-5-252996A beschriebenen Verfahren sollen alle Zellen aus einer Lösung auf einen Filter gebracht werden. Dies gilt ebenfalls für das in JP-07143898A beschriebene Verfahren.

In Hirte, H. W. et al., Gynecologic Oncology, 44, 1992, 223-226, ist die Isolierung von Zellklumpen aus Ascites-Flüssigkeit von Patienten mit Ovarialkarzinomen beschrieben. Es handelt sich hierbei um große Zellklumpen, die von einem Ovarialkarzinom in fortgeschrittenem Stadium stammen und unter den dort gegebenen Umständen lokal in hoher Konzentration anzutreffen sind.

Dagegen kann die Isolierung von disseminierten Krebszellen bekanntermaßen mit Verfahren gelingen, bei denen die Krebszellen so markiert werden, daß sie von nicht entarteten Zellen zu unterscheiden sind und aufgrund dessen aussortiert werden können. Neben der klassischen Säulentechnik werden zu diesem Zweck beispielsweise auch sogenannte "Cross-Flow" und "Through-Flow" Filter beschrieben, die mit spezifischen Liganden beladen sind (z.B. in der WO 96/06158, die allerdings nicht auf Tumorzellen abstellt).

Die überwiegende Anzahl derartiger Verfahren beruht auf einer antigenspezifischen Immunadsorption (vergleiche beispielsweise Rye, P.D. et al., The American Journal of Pathology, 150,1997, 99-106). Antikörper gegen bestimmte tumorspezifische oder epitheliale Zelloberflächenmoleküle werden beispielsweise mit fluoreszierenden und insbesondere magnetischen Markem versehen. Diese Verfahren haben den Nachteil, daß durch eine Quervernetzung der Oberflächenantigene unkalkulierbare Effekte, wie Apoptose, Anergie, Aktivierung und weitere Zustandsänderungen der Zellen auftreten können. Derartige Effekte können das Bild einer anschließenden Charakterisierung der isolierten Krebszellen drastisch verändern. So kann beispielsweise das Expressionsprofil einer Zelle innerhalb weniger Minuten beeinflußt werden. Verständlicherweise kann in solchen Fällen nicht ausgeschlossen werden, daß die so erhaltenen Analyseergebnisse Scheineigenschaften widerspiegeln, welche die disseminierten Krebszellen in der Körperflüssigkeit vor ihrer Isolierung nicht aufwiesen. Dies wäre aber wünschenswert. Ferner ist von Nachteil, daß die anhaftenden Antikörper nicht oder nur mit ungünstigen Auswirkungen auf die Zelle entfernt werden können.

Richten sich die Antikörper gegen intrazelluläre Bestandteile, ist sogar eine Fixierung und Perforierung der Zelle erforderlich, was deren Tod zur Folge hat. Unter derartigen Umständen sind sogenannte Bioassays, die mit lebenden und insbesondere vermehrungsfähigen Zellen arbeiten, mit großen Schwierigkeiten behaftet oder gar unmöglich. Ein weiterer Nachteil der Aufreinigung über Antikörper liegt in der Kreuzreaktivität bestimmter Epitope, so daß auch "normale" Zellen mitisoliert werden können. Außerdem können durch Clusterbildung mit Blutbestandteilen, z.B. Blutplättchen, Fibrin und ähnlichem, isolationstechnisch wichtige Epitope zumindest teilweise verdeckt werden.

Die Anforderungen, die an ein Verfahren zur Isolierung von Krebszellen im Rahmen der Identifizierung und Charakterisierung disseminierter Krebszellen gestellt werden müssen, sind hoch. Neben der üblicherweise im Hinblick auf das Verfahrensprodukt geforderten hohen Ausbeute und Reinheit, ist es im vorliegenden Fall vor allem die Authentizität der isolierten Krebszellen, welche die Brauchbarkeit eines derartigen Isolierungsverfahrens bestimmt. Die Krebszellen sollten möglichst unverändert aus der Körperflüssigkeit isoliert werden können, also nicht mit isolationstechnisch bedingten Konstrukten, wie Glasbeads behaftet sein. Sie sollten ex vivo kultiviert werden können und in Bio-Assays ein zuverlässiges Bild ihres ursprünglichen Zustandes in der Körperflüssigkeit wiedergeben.

Ein Verfahren, das diesen Anforderungen gerecht wird, nutzt die entscheidenden Vorteile aus, die sich aus der Identifizierung und vor allem Charakterisierung von Krebszellen aus Körperflüssigkeiten ergeben. Vergleicht man Zellen aus Primärtumorgewebe mit entsprechenden disseminierten Tumorzellen, so weisen die disseminierten Tumorzellen in der Regel genetische und physiologische Merkmale auf, die sich von denen des Primärtumors unterscheiden, beispielsweise durch klonale Selektion aus diesen entstehen können. Diese Merkmale liefern wichtige, gegebenenfalls zusätzliche Informationen für die Diagnose, Prognose, Prädiktion und weitere onkologische Fragestellungen.

Aufgabe der vorliegenden Erfindung war es daher, ein schonendes Verfahren zur Isolierung von Krebszellen aus zellhaltigen Körperflüssigkeiten zur Verfügung zu stellen, das den Zustand dieser Krebszellen nicht oder nur unwesentlich beeinflußt.

Überraschenderweise wurde gefunden, daß es gelingt, Krebszellen aus zellhaltigen Körperflüssigkeiten durch einen größen- und/oder gestaltabhängigen Trennvorgang zu isolieren.

Gegenstand der vorliegenden Erfindung ist daher ein Verfahren zur Isolierung von Krebszellen aus zellhaltigen Körperflüssigkeiten, das dadurch gekennzeichnet ist, daß man die zellhaltige Körperflüssigkeit oder Teile davon durch ein Sieb mit einer Maschen- oder Porenweite von etwa 15 bis 30 µm oder ein Sieb mit einem absoluten oder nominalen Rückhaltevermögen von etwa 15 bis 30 µm führt und die zurückgehaltene Zellfraktion gewinnt.

Unter Isolierung versteht man erfindungsgemäß jegliche Anreicherung eines zu isolierenden Bestandteils aus einem Gemisch, das diesen neben wenigstens einem anderen Bestandteil enthält. Das Ergebnis der Isolierung kann also durchaus auch ein weiteres Gemisch sein, das aber im Vergleich zum ursprünglichen Gemisch den zu isolierenden Bestandteil im Verhältnis zu wenigstens einem anderen Bestandteil in höherer Konzentration enthält.

Der Begriff Krebszelle steht erfindungsgemäß für eine Zelle, die eine oder mehrere mit Krebs, also Entartung im allgemeinen Sinn, in Zusammenhang stehende Modifikation aufweist. Grundlage dieser Definition ist die Vorstellung, daß es sich bei der Entstehung von Krebs um einen kontinuierlichen Veränderungsprozeß handelt. Beispielsweise bedarf es in der Regel mehrerer Veränderungen insbesondere des genetischen Materials bzw. der Expression des genetischen Materials von Zellen auf dem Weg von einer Normalzelle zu einer Krebs- und insbesondere einer Tumorzelle. Der Begriff Krebszelle umfaßt daher auch Vorstufen von Krebs- und insbesondere Tumorzellen mit krebsartigen bzw. tumorösen Modifikationen. Disseminierte Tumorzellen, also Zellen, die sich vom Primärtumor abgelöst haben und in Körperflüssigkeiten zirkulieren, gelten nicht als eigentlicher Tumor im medizinischen Sinn, sie stellen aber Krebszellen im erfindungsgemäßen Sinn dar. Erfindungsgemäß gehören zu disseminierten Tumorzellen auch mikrometastasierte und metastasierende Tumorzellen, sofern sich diese in einer erfindungsgemäßen Körperflüssigkeit befinden.

Unter Sieb versteht man erfindungsgemäß ein Material, das einen größen- und/ oder gestaltabhängigen, d.h. auf Zellgröße, Verformbarkeit, Aggregat- oder Clusterbildung basierenden, Trennvorgang ermöglicht. In Bezug auf die Isolierung von Krebszellen versteht man unter Sieb also ein Trennmittel, mit dem sich Krebszellen von Nichtkrebszellen trennen lassen. Der Siebvorgang führt zu einer räumlichen Aufteilung von Krebszellen und Nichtkrebszellen; zur Bildung von wenigstens zwei Zellfraktionen; zur bevorzugten Zuordnung von Krebszellen zu wenigstens einer Zellfraktion und zur bevorzugten Zuordnung von Nichtkrebszellen zu wenigstens einer weiteren Zellfraktion; gegebenenfalls zur im wesentlichen ausschließlichen Zuordnung von Krebszellen zu wenigstens einer Zellfraktion.

In der Regel findet man im Siebrückstand - je nach Krebszellgehalt der Körperflüssigkeit - bis zu 100 Krebszellen pro ml Körperflüssigkeit. Im Vergleich zu den in der Körperflüssigkeit vorhandenen Nichtkrebszellen - im Falle von Blut insbesondere den mononukleären Zellen - erreicht man in der Regel Anreicherungsfaktoren von 10⁵ und höher, vorzugsweise von mindestens 5 x 10⁵, bevorzugter von mindestens 10⁶ und insbesondere von wenigstens 5 x 10⁶. Diese Faktoren lassen sich gegebenenfalls durch weitere dem Siebvorgang vor- und/oder nachgeschaltete Trennvorgänge erhöhen. So werden bei der üblicherweise an Blut zunächst vorgenommenen Abtrennung der MNC-Fraktion, z.B. durch Dichtegradientenzentrifugation, Anreicherungsfaktoren von etwa 10² erreicht.

Im Hinblick auf die anschließende Verwendung der Krebszellen können an das Verhältnis von Krebszellen zu Nichtkrebszellen unterschiedlich hohe Anforderungen gestellt sein. Sensitive Untersuchungsmethoden, wie die p53-Analytik, erfordern beispielsweise ein Verhältnis von wenigstens einer Krebszelle zu 1000 Nichtkrebszellen während weniger sensitive Untersuchungsmethoden, wie die LOH-Analytik, ein Verhältnis von wenigstens 1:1 erfordern.

Erfindungsgemäß kann man ein Sieb wählen, das Nichtkrebszellen der zellhaltigen Körperflüssigkeit gerade noch passieren läßt. Ebenfalls geeignet ist ein Sieb, das auch größere Partikel als Nichtkrebszellen passieren läßt, Krebszellen oder wenigstens ein Teil der Krebszellen hingegen zurückhält. Unter Berücksichtigung eines größen-und/ oder gestaltabhängigen Trennvorgangs kann die Wahl des Siebes, insbesondere in Abhängigkeit von der verwendeten Körperflüssigkeit, optimiert werden.

Bei dem Sieb handelt es sich in der Regel um ein flächiges Gebilde mit Öffnungen, die auch als Maschen bezeichnet werden. Neben flächigen Materialien sind auch poröse Körper, beispielsweise Filter, oder membranartige Materialien geeignet. Voraussetzung ist allerdings, daß die Porengröße hinreichend definiert ist.

Die Größen der Öffnungen eines Siebes liegen in der Regel innerhalb eines bestimmten Bereichs. Die Angabe von Unter- und Obergrenze bedeutet nicht, daß auch Öffnungen mit genau diesen Grenzwerten auf dem Sieb vorhanden sein müssen. Wohl aber bedeutet eine solche Angabe, daß Öffnungen, deren Größe diese Werte unter- bzw. überschreitet, auf dem Sieb nicht vorhanden sind. Vorzugsweise liegen die Größen der Öffnungen eines Siebes innerhalb eines eng definierten Bereichs. Idealerweise sind sie im wesentlichen einheitlich. Die Größen der Öffnungen eines Siebes werden im folgenden als Maschenweite oder Porengröße/Porenweite bezeichnet.

Ein erfindungsgemäß einsetzbares Sieb weist in der Regel eine Maschenweite bzw. Porenweite von 15, insbesondere 17, bis 30 µm und besonders bevorzugt von etwa 20 µm auf. Gemeint sind sowohl Siebe, deren Maschen- bzw. Porenweite eine gewisse Verteilung innerhalb der vorstehend genannten Bereiche aufweist, als auch Siebe mit einer im wesentlichen einheitlichen Maschen- bzw.

Porenweite, deren Wert innerhalb der vorstehend genannten Bereiche liegt.

Siebe mit gleichmäßiger Maschen- bzw. Porenweite erlauben Angaben zum absoluten Rückhaltervermögen, d.h. der Größe der gerade noch passierbaren Partikel. Siebe mit unregelmäßiger Poren- bzw. Maschenweite hingegen gestatten lediglich die Angabe von nominalen Rückhaltevermögen, wonach 98% aller Partikel, die größer als dieses nominale Rückhaltevermögen sind, zurückgehalten werden.

Erfindungsgemäß einsetzbare Siebe weisen in der Regel ein absolutes bzw. nominales Rückhaltevermögen von 15, insbesondere 17, bis 30 µm und insbesondere von etwa 20 µm auf.

Die Wahl des Materials für die sowohl flächige Gebilde, wie Sieb- oder Membranfilter, als auch poröse Körper wie Tiefenfilter umfassenden erfindungsgemäßen Siebe ist von untergeordneter Bedeutung. Zu nennen sind vor allem faserbildende Materialien, insbesondere organische Polymere oder anorganische Fasern, und Bildner mikroporöser Matrices organischen oder anorganischen Ursprungs. Beispielsweise können Harze, Gele, Granulate, sinterfähige Materialien, Glase, Keramiken, Molekularsiebe, z.B. Zeolithe, etc. verwendet werden. Organische Polymere können natürlichen, d.h. tierischen oder pflanzlichen Ursprungs, halbsynthetisch oder vollsynthetisch sein. Zu nennen sind beispielsweise keratinhaltige Strukturen, Haare, z.B. Kamelhaar, Wolle, Angora, Seide, cellulosehaltige Strukturen, Baumwolle, Flachs, Hanf, Jute etc. Erwähnenswert sind halbsynthetische Polymere auf Cellulosebasis, beispielsweise Celluloseester, insbesondere Celluloseacetat und Nitrocellulose, sowie gemischte Celluloseester. Zu brauchbaren vollsynthetischen Polymeren gehören Polyolefine, wie Polyethylen (PE), Polypropylen (PP), Cyclopolyolefine, Polyamide, wie Nylon (GRILON, GRILAMID), Nylon 6, Nylon 6,6, Nylon 11, Nylon 12, Copolymamide (GRILON C), Aramide, Poly(p-phenylenteraphthalamid) (KEVLAR), Polyester, wie Poly(alkylentheraphthalat), insbesondere Poly(ethylentheraphthalat) (PETP), Acrylpolymere, wie Polyacrylonitril (DRALON) und Acrylate, Vinylpolymere, wie Poly(vinylchlorid) (PVC), Poly(vinylalkohole), Polyesterether, wie Polyetheretherketon (PEEK), Polyurethane, Epoxide, Fluorkohlenstoffpolymere, wie Poly(vinylidenfluorid) (PVDF), Poly(tetrafluorethylen) (PTFE, TEFLON), Polyhexafluorethylenpropylen-Copolymer (FEP), Polyethylentetrafluorethylen-Copolymer (ETFE, AFLON), Polyethylenchlortrifluorethylen-Copolymer (ECTFE, HALAR), Polycarbonate, wie PCTE, Polyphenylensulfide (PPS), Polyethersulfone, etc. Glase, insbesondere Borosilikate und Siliciumdioxide, Silicium, Metalle, Keramiken, Kohlenstoff und Asbest sind beispielsweise als anorganische Faserbildner zu nennen. Auch Gemische aus den vorstehend genannten Materialien können verwendet werden. Erforderlichenfalls können die Materialien, insbesondere die aus ihnen bestehenden Fasern, modifiziert, beispielsweise metallisiert, hydrophobiert, hydrophiliert, z.B. mit Polyvinylpyrrolidon, mit Stützstrukturen versehen, vernetzt, oder mit Bindemitteln, z.B. Acrylaten oder Melaminharzen, umgeben sein.

Siebe aus Iösungsmittelbeständigem Material sind für bestimmte Ausführungsformen der Erfindung von Vorteil. Bevorzugt werden lösungsmittelbeständige Kunststoffe, wie Polypropylen, Polytetrafluorethylen, hochfluorierte Polymere, Vinylidenfluorid, Aminoplaste, insbesondere Polyethylen. Auch Metalle, Gläser und andere mineralische Werkstoffe sowie bestimmte Naturfasern sind grundsätzlich geeignet.

Die Herstellung derartiger Siebe liegt im Bereich fachmännischen Könnens, z.B. können Webverfahren, Ätzverfahren, sowohl trocken als auch naßchemisch, die Laserstrukturierung, z.B. RMPD-Maskentechnik, photolithographische Verfahren, LIGA, etc. eingesetzt werden.

Faserbildende Materialien können verschiedene Faserstrukturen aufweisen. Zu nennen sind beispielsweise glatte, kantige, wellige, zerfranste, fasrige und ähnliche Oberflächenformen; kreisrunde, elliptische, knochenartige, gezackte, gelappte und ähnliche Querschnittsformen; geradlinige, gekrümmte, helikale und ähnliche Axialformen (Textur); bei mehr als einer Komponente unterschiedliche Querschnittsverteilungen, z.B. bei Bikomponentfasern Anordnungen der beiden Komponenten Seite-an-Seite, als Ummantelung, Beschichtung, Kern-Schale-Typ oder Mehrkernanordnungen ("Inseln-im-Meer").

Vorzugsweise handelt es sich um Gewebe aus Fäden, Fasern, Filamenten oder Bündeln davon mit Öffnungen verschiedenster Geometrie. Mono- und/oder multifilamente Fasern können verwendet werden. Mögliche Webstrukturen sind nicht zuletzt aus dem Textilbereich bekannt. Vorteilhaft sind Webestrukturen, bei denen der prozentuale Anteil der offenen Fläche (Gesamtfläche sämtlicher Öffnungen) einen problemlosen Siebvorgang ermöglicht. 1:1- und 2:1-Strukturen werden in der Regel bevorzugt. Auch für die Oberfläche der Fäden, Fasern, Filamente oder Bündel kommen verschiedene Ausführungen in Frage. So kann die Oberfläche gleichmäßig oder ungleichmäßig, beispielsweise glatt, kantig, wellig, zerfranzt oder haarig sein. Ferner sind auch perforierte Platten geeignet, wobei ebenfalls Öffnungen verschiedenster Geometrie und Anordnung möglich sind.

Das Sieb kann ein- oder mehrschichtig sein. Für bestimmte erfindungsgemäße Ausführungsformen werden einschichtige Siebe bevorzugt. Es können auch mehrere, ggf. unterschiedliche Siebe hintereinander angeordnet werden.

Zweckmäßigerweise ist das Sieb in einer Vorrichtung angeordnet, die es gestattet, die zellhaltige Flüssigkeit durch das Sieb zu führen und den Siebdurchgang aufzufangen. Das Sieb sollte dieser Vorrichtung auch entnommen werden können, um zusammen mit dem Siebrückstand weiteren Verfahrensschritten zugeführt werden zu können. Falls erforderlich, können auch Mittel zum Anlegen von Druck oder Unterdruck vorgesehen sein, um den Siebvorgang zu erleichtern. Geht dem Siebvorgang eine vorbereitende Aufarbeitung der zellhaltigen Körperflüssigkeit voraus, so kann es zweckmäßig sein, Vorrichtungen und Mittel zur Durchführung des Siebvorganges an Vorrichtungen und Mittel zur Durchführung der Aufarbeitung zu koppeln, wobei der Siebvorgang der Aufarbeitung nachgeschaltet wird. Darüber hinaus kann der Fachmann weitere Maßnahmen vorsehen, um den üblicherweise im Bereich der Biochemie und Molekularbiologie gestellten Anforderungen, wie Temperatur und Sterilität, zu genügen.

Das erfindungsgemäße Verfahren kann auf all diejenigen zellhaltigen Körperflüssigkeiten angewendet werden, die Krebszellen und insbesondere disseminierte und mikrometastasierte Krebszellen aufweisen. Dies sind sowohl native Körperflüssigkeiten, die dem Körper entnommen werden oder von diesem ausgeschieden werden, als auch nichtnative Flüssigkeiten, insbesondere Waschflüssigkeiten, die Zellen aus dem Körper und insbesondere bestimmten Körperteilen und Organen enthalten. Man kann z.B. die Flüssigkeit in geeigneter Weise dem Körper zunächst zuführen und dann wieder entnehmen. Auch können native mit nichtnativen Flüssigkeiten versetzt sein. Zu nennen sind beispielsweise Lymphe, Urin, Sputum, Fruchtwasser, Punktate, Waschflüssigkeiten von Organen, z.B. Colon-, Lungen-, Bronchiallavage oder Blasenspülflüssigkeit, Fäces, und insbesondere Knochenmark und Blut. Es kann sich um Körperflüssigkeiten verschiedener Species handeln, beispielsweise von Säugern, insbesondere Menschen, Labor- und Versuchstieren, wie Mäusen, Ratten, Kaninchen, Meerschweinchen, etc. Derartige Körperflüssigkeiten können dem Siebvorgang direkt zugeführt werden. Vielfach ist es allerdings von Vorteil, die zellhaltige Körperflüssigkeit zunächst einer vorbereitenden Aufarbeitung zu unterziehen. So kann man beispielsweise zelluläre von nicht-zellulären Bestandteilen trennen. Auch die zellulären Bestandteile können gegebenenfalls noch weiter aufgetrennt werden, indem man beispielsweise eine zellhaltige Fraktion isoliert, in der bekanntermaßen Krebszellen mitenthalten sind. Zu diesem Zweck bieten sich vor allem die eingangs beschriebenen physikalischen Trennverfahren, wie die Dichtegradientenzentrifugation, an.

Werden Krebszellen aus Blut isoliert, ist es erfindungsgemäß bevorzugt, zunächst Zellen des weißen Blutbildes durch Dichtegradientenzentrifugation abzutrennen. Krebszellen findet man vor allem in der Fraktion, die auch mononukleäre Zellen enthält, so daß diese Fraktion bevorzugt dem anschließenden Siebvorgang zugeführt wird.

Weiterhin ist es auch möglich, die Krebszellen in der Zellsuspension vor dem Siebvorgang zu verändern, beispielsweise zu markieren, Partikel anzuhängen, eine Aggregation und/oder Clusterbildung auszulösen, z.B. durch geeignete Antikörper, Enzyme, Lectine, andere Liganden und/oder Rezeptoren oder kreuzvernetzende Reagenzien, zu fixieren und andere definierte Zustände zu induzieren.

Um dem Siebvorgang zugeführt werden zu können, sollten die zuvor aus einer Körperflüssigkeit isolierten zellhaltigen Fraktionen (Isolate) in Suspension vorliegen. Als Suspensionsmedium wählt man vorteilhafterweise einen Puffer oder ein Kulturmedium. Das Suspensionsmedium sollte möglichst keine Auswirkungen auf die interessierenden Eigenschaften der Zellen haben.

Bevor man die zellhaltige Körperflüssigkeit oder Isolate davon durch ein Sieb führt, ist es für die spätere Auswertung von Vorteil, Aliquots an zellhaltiger Flüssigkeit abzunehmen. Derartige Proben können als Bezugsgröße für die an Siebruck-stand und Siebdurchlauf ermittelten Meßwerte dienen. Dies erlaubt einen Abgleich gegen Nichtkrebszellen, z.B. als CD-45-positive Zellen isolierte Lymphozyten als Patienteneigene interne Kontrolle, wodurch Analysen an den isolierten Krebs-zellen mit besonderer Sensitivtät durchgeführt werden können.

Der Siebvorgang ist beendet, wenn die gesamte zellhaltige Flüssigkeit das Sieb passiert hat. Es kann sich ein Waschvorgang anschließen, bei dem weitere Flüssigkeit, vorzugsweise Puffer oder Kulturmedium, durch das Sieb geführt wird. Die Waschflüssigkeit kann zu dem zuvor gewonnenen Siebdurchlauf gegeben oder auch getrennt davon gesammelt und gegebenenfalls verworfen werden.

Die auf dem Sieb zurückgehaltene Zellfraktion kann direkt der sich anschließenden Verwendung, beispielsweise der Charakterisierung der Zellen, insbesondere der Krebszellen, oder der Aufbewahrung zugeführt werden. Vorteilhafterweise werden die Zellen, insbesondere die Krebszellen, allerdings zunächst vom Sieb abgelöst. Je nach Art der anschließenden Verwendung kann man zu diesem Zweck verschiedene Vorgehensweisen wählen.

Eine Möglichkeit besteht darin, die auf dem Sieb zurückgehaltenen Zellen, insbesondere die Krebszellen, abzuwaschen, indem man eine geeignete Flüssigkeit in entgegengesetzter Richtung durch das Sieb führt und die zellhaltige Waschflüssigkeit gewinnt. Die abgelösten Zellen können dann gewünschtenfalls aus der Zellsuspension pelletiert werden.

Auch ist es möglich, die am Sieb anhaftenden Zellen, insbesondere die Krebszellen, unter Krafteinwirkung abzulösen. Schwerkraft, Zentrifugalkraft und elektrische Kräfte können eingesetzt werden. Sedimentation, Zentrifugation, Elektrophorese, Dielektrophorese, Verwendung einer sogenannten optischen Pinzette, Elektroosmose, und ähnliche Verfahren sind dem Fachmann zu diesem Zweck geläufig. Dies gelingt beispielsweise, indem man das Sieb so in ein geeignetes Medium, in der Regel eine Flüssigkeit gibt, daß die Zellen durch Zentrifugation pelletiert werden können. Auf diese Weise können die Zellen direkt in ein Medium überführt werden, das für die nachfolgende Verwendung geeignet ist.

Eine weitere Möglichkeit, die auch zum Ablösen der Zellen, insbesondere der Krebszellen, vom Sieb führt, durch die diese Zellen allerdings gleichzeitig zerstört werden, besteht darin, das Sieb samt anhaftender Zellen den der Gewinnung von Zellbestandteilen, z.B. Nukleinsäuren und Proteinen, dienenden Verfahrenzuzuführen. Sofern derartige Maßnahmen den Einsatz organischer Lösungsmittel erfordern, beispielsweise die in diesem Bereich vielfach zur Isolierung von Gesamt-RNA, DNA und Proteinen verwendeten Guanidinisothiocyanat und Phenol enthaltenden Lösungen, benutzt werden, sind Siebe aus lösungsmittelbeständigen Materialien bevorzugt.

Das erfindungsgemäße Verfahren führt zur Isolierung von Krebszellen aus zellhaltigen Körperflüssigkeiten. Unter Isolierung von Krebszellen im erfindungsgemäßen Sinne versteht man die Herstellung von Krebszellen enthaltenden Zellgemischen aus Körperflüssigkeiten, wobei das Verhältnis von Krebszellen zu Nichtkrebszellen in den hergestellten Zellgemischen größer ist als in den ursprünglichen zellhaltigen Körperflüssigkeiten. So bedeutet Isolierung die Anreicherung von Krebszellen in zellhaltigen Fraktionen von Körperflüssigkeiten. Es werden zellhaltige Fraktionen mit erhöhtem Krebszellanteil hergestellt und gewonnen.

Vorzugsweise handelt es sich bei den zellhaltigen Fraktionen um Zellgemische mit einem Krebszellanteil von wenigstens 50%, bevorzugter von wenigstens 80% und insbesondere von wenigstens 90%.

Isolierung im erfindungsgemäßen Sinn bedeutet auch die Gewinnung von Krebszellen, die im wesentlichen frei von Nichtkrebszellen sind. Diese können in Form von Krebszellgemischen gewonnen werden, die in der Regel polyklonal sind, durchaus aber auch gemeinsame Merkmale aufweisen können. Es können allerdings auch Fraktionen dieser Krebszellgemische und auch Einzelzellen gewonnen werden, die (weitere) gemeinsame Merkmale aufweisen und/oder gegebenenfalls sogar monoklonal sind. Dies bedarf unter Umständen einer Subtypisierung der erfindungsgemäß aus zellhaltigen Körperflüssigkeiten isolierten, d.h. angereicherten Krebszellen, anhand von Stratifizierungsparametern (z.B. immunologisch) und/oder einer weiteren Aufreinigung. Zu nennen sind hier beispielsweise die Laser-Mikrodissektion, Mikromanipulation, Dielektrophorese, Elektroosmose, Optische Pinzette, FACS und ähnliches nach spezifischer Adressierung.

Isolierung im erfindungsgemäßen Sinne bedeutet auch ein Abtrennen von Krebszellen aus zellhaltigen Präparaten, insbesondere Körperflüssigkeiten oder Teilen davon, d.h. eine Reduktion des Krebszellanteils in zellhaltigen Präparaten, beispielsweise aus Blut oder Blutbestandteilen, vorzugsweise extrakorporal, aus Stammzellpräparaten oder sonstigen Reinfusions-, Transfusions- und Transplantationspräparaten.

Das erfindungsgemäße Verfahren kann somit auch zur, gegebenenfalls vollständigen, Entnahme kontaminierender Krebszellen aus Zellgemischen, d.h. zellhaltigen Präparaten, insbesondere Körperflüssigkeiten oder aus Isolaten davon, angewendet werden (Depletion).

Gegenstand der vorliegenden Erfindung sind somit auch Verfahren zur Entnahme, insbesondere zur extrakorporalen Elimination, von Krebszellen, insbesondere von Tumorzellen, aus zellhaltigen Präparaten, insbesondere Körperflüssigkeiten oder Teilen davon, und auch Verfahren zur Depletion von Stammzellpräparaten oder sonstiger Reinfusions- und/ oder Transfusionspräparaten zur Reduktion des Krebszellgehaltes. Zweck dieser Verfahren ist insbesondere die Rezidivprophylaxe. So stellen disseminierte Tumorzellen in Körperflüssigkeiten und anderen Zellpräparaten einen erheblichen Risikofaktor für die Entwicklung von Rezidiven bzw. Metastasen dar. Die Konzentration und die Zusammensetzung der Zellen beeinflußt - neben ihrer genetischen Disposition - die Wahrscheinlichkeit, mit der sich Metastasen entwickeln. Die Verringerung der Konzentration an Krebszellen, insbesondere von disseminierten Tumorzellen, in diesen Körperflüssigkeiten durch ein Verfahren, mit dem diese extrakorporal entfernt werden, reduziert die Wahrscheinlichkeit für ein Rezidiv. Beispielsweise erhalten viele Tumorpatienten nach Bestrahlung und/oder Chemotherapie autologe Zellisolate. Diese Zellen, beispielsweise Apherese-Produkte, isolierte Stammzellen und dergleichen, können mit dem erfindungsgemäßen Verfahren auf schonende Weise von Krebszellen befreit werden. Das Risiko eines Rezidivs durch kontaminierende Krebszellen kann so reduziert werden.

Die vorliegende Erfindung betrifft daher auch die Verwendung des Verfahrens zur Behandlung des menschlichen oder nicht-menschlichen tierischen Körpers mit dem therapeutischen Ziel der Rezidivprophylaxe.

Die vorliegende Erfindung betrifft auch den verfahrensgemäß gewonnenen Siebrückstand sowie daraus erforderlichenfalls unter Verwendung weiterer an sich bekannter Maßnahmen abgeleitete Fraktionen, insbesondere Zellgemische, Krebszellgemische, Krebszellklone und/oder Krebszellbestandteile. Gegenstand sind also auch aus Körperflüssigkeiten mit einem der vorstehend beschriebenen Verfahren erhältliche - umfassend als Krebszellmaterial bezeichnete - Zellgemische, Krebszellgemische, Krebszellklone, daraus etablierte Zellinien und/ oder Krebszellbestandteile.

Die aus Körperflüssigkeiten abgeleiteten Zellgemische weisen einen Krebszellanteil von wenigstens 50%, vorzugsweise von wenigstens 80% und insbesondere von wenigstens 90% auf. Bei dem jeweiligen Restanteil kann es sich um Nichtkrebszellen aus der betreffenden Körperflüssigkeit handeln.

Besonders bevorzugt sind aus Körperflüssigkeiten abgeleitete Krebszellgemische, die nur geringe Mengen und vorzugsweise keine Nichtkrebszellen enthalten. Diese Gemische sind in der Regel polyklonal. Obwohl polyklonal, so können sie doch im Hinblick auf wenigstens ein Merkmal, beispielsweise einer bestimmten genomischen Disposition oder eines Expressionsparameters, homogen sein. Bevorzugt werden Krebszellgemische, die in Bezug auf wenigstens 1, 2, 3, 4, 5, 10 oder 15 analytisch zu bestimmende Parameter im wesentlichen homogen sind. Zu diesen Bestimmungen zählen krebsnachweisende Analysen, tumorbiologisch relevante Analysen zur Messung zellphysiologischer Parameter, Analysen pharmakologisch relevanter Parameter, Bioassays, cytologische Analysen, und ähnliche Verfahren, auf die nachfolgend noch näher eingegangen wird.

Insbesondere bevorzugt sind Krebszellklone, d.h. einzelne Krebszellen oder monoklonale Krebszellinien.

Ausgehend von den vorstehend beschriebenen Zellgemischen, Krebszellgemischen und Krebszellklonen, vor allem aus den Krebszellgemischen und insbesondere den Krebszellklonen, können Zellinien etabliert werden. Es kann sich sowohl um Kurzzeit- als auch Langzeit-Zellinien handeln. Diese Zellinien können in fachmännischer Weise etabliert werden, eine geeignete Methodik, beispielsweise im Hinblick auf die Wahl des Kulturmediums oder der Kulturbedingungen sowie der Aufbewahrung bzw. Konservierung, ist dem einschlägigen Fachmann bekannt. Gegebenenfalls kann auf Kenntnisse zu bereits etablierten Krebszellinien, wie HeLa, zurückgegriffen werden.

Zu den Zellbestandteilen, die aus den vorstehend beschriebenen zellhaltigen Präparaten abgeleitet sind, zählen beispielsweise Zellysate sowie Fraktionen davon, also bestimmte Zellbestandteile, wie Nukleinsäuren, Proteine, etc., die in fachmännischer Weise aus den zuvor gewonnenen zellhaltigen Präparaten isoliert werden.

Erfindungsgemäß gelingt es, aus Körperflüssigkeiten isolierte Krebszellen zur Verfügung zu stellen, die frei vom verwendeten Trennmittel sind. Sie sind also insbesondere frei von herkömmlicherweise zu Isolationszwecken verwendeten Liganden, wie Antikörpern, Lektinen, etc. Die erfindungsgemäßen Krebszellen befinden sich daher in einem biologischen Zustand und nicht in einem künstlichen Zustand, wie er üblicherweise im Zuge ihrer Isolierung, z.B. durch Fixierung und/oder Markierung, herbeigeführt wird. Ein biologischer Zustand im erfindungsgemäßen Sinne ist daher ein Zustand, den die betreffende Zelle insbesondere im Hinblick auf Physiologie, Morphologie und/ oder Expressionsprofil in einer Körperflüssigkeit eines menschlichen oder nicht-menschlichen tierischen Individuums annehmen kann. Zur Beschreibung dieses biologischen Zustandes sind vor allem Parameter in Bezug auf Zellzyklus, Aktivierung, Proliferation, Apoptose und ähnliches von Bedeutung. Diese werden durch den erfindungsgemäßen Isolationsvorgang im wesentlichen nicht verändert. Ein biologischer Zustand kann somit durch eine oder mehrere der nachfolgend beispielhaft genannten Eigenschaften gekennzeichnet sein: vital, teilungsfähig, proliferativ, kultivierbar, präapoptotisch, apoptotisch, tot, vereinzelt, aggregiert, clusterbildend, etc.

Gegenstand der vorliegenden Erfindung sind daher auch aus Körperflüssigkeiten isolierte - ebenfalls unter den erfindungsgemäßen Begriff Krebszellmaterial zu fassende - Krebszellen, insbesondere disseminierte Tumorzellen, deren Zustand biologisch, vorzugsweise vital, ist, gegebenenfalls im Gemisch mit Nichtkrebszellen, sowie daraus etablierte Zellinien. Isoliert bedeutet in diesem Zusammenhang, daß das Verhältnis von Krebszellen zu Nichtkrebszellen größer ist als in der ursprünglichen Körperflüssigkeit. Bevorzugt ist ein Krebszellanteil von wenigstens 50%, vorzugsweise von wenigstens 80% und insbesondere von wenigstens 90%. Ganz besonders bevorzugt sind im wesentlichen reine Krebszellen, die in vorteilhafter Ausgestaltung die Merkmale der vorstehend beschriebenen Krebszellgemische, Krebszellklone und der daraus etablierten Zellinien aufweisen.

Einen besonderen Aspekt der vorliegenden Erfindung bilden erfindungsgemäße Krebszellen, die durch eine in quantitativer und/oder qualitativer Hinsicht bestimmte Kombination von Parametern (Muster) gekennzeichent sind. Die musterbildenden Parameter betreffen vor allem genomische Dispositionen und/oder Expressionsprofile. Besondere Krebszellen ergeben sich beispielsweise unter Bezugnahme auf die in der WO 99/10528 offenbarten Kombinationen bestimmter Gene zur Vornahme von Multiparameter-Expressionsanalysen sowie genomischer Untersuchungen auf Onkogene und/oder mutierte Tumorsuppressorgene. Zu nennen sind also beispielsweise Krebszellen, die CEA und CK20, gegebenenfalls in Kombination mit tumorspezifischen Splice-Varianten des MUC1-Gens; oder MAGE3 und Tyrosinase gegebenenfalls in Kombination mit Muc18 exprimieren; und/oder Krebszellen mit wenigstens zwei der unter p53-Mutationen und/oder - LOH's, erb-B2-Amplifikationen, c-myc-Amplifikationen und K-ras-Mutationen ausgewählten genomischen Dispositionen gegebenenfalls in Kombination mit LOH's der Gene RB, APC, DCC und/ oder DPC4; und/ oder Krebszellen, die Maspin und/ oder den Progesteron-Rezeptor, gegebenenfalls in Kombination mit βhCG, dem Östrogenrezeptor und/ oder SCCA; PSM und/oder PSA gegebenenfalls in Kombination mit hK2; Gastrin gegebenenfalls in Kombination mit GIP und/ oder Motilin; oder SP-A und SP-C gegebenenfalls in Kombination mit β-hCG exprimieren; und/ oder bFGF, bFGF-R, VEGF-R1 und/ oder VEGF-R2 gegebenenfalls in Kombination mit VEGF; MMP's, insbesondere MMP2; und/oder TIMP's, insbesondere TIMP3 exprimieren; und/oder FAS-L und FAS-R; gegebenenfalls Cycline, insbesondere Cyclin B, D und E in zellzyklusspezifischen Verhältnissen, Ki67, bax und/ oder bcl-2 exprimieren. Mit Expression kann eine qualitative Expression und auch eine im Vergleich zu Nichtkrebszellen quantitativ erhöhte oder verminderte Expression gemeint sein. Die vorstehend beschriebenen Muster können auch die aus den Krebszellen etablierten Zellinien und abgeleitete Zellbestandteile kennzeichnen.

Natürlich können an den isolierten Krebszellen gezielte Eingriffe vorgenommen werden, die den biologischen Zustand in einen künstlichen Zustand überführen, beispielsweise indem man sie fixiert, markiert, beispielsweise radioaktiv, mit PET-Labeln, NMR-Sonden, etc., oder einen anderen Zustand induziert, der durchaus auch einem biologischen Zustand entsprechen kann.

Die vorliegende Erfindung betrifft daher auch aus Körperflüssigkeiten isolierte - ebenfalls unter den erfindungsgemäßen Begriff Krebszellmaterial zu fassende - Krebszellen, insbesondere disseminierte Tumorzellen, deren Zustand ein nach Isolierung ausgehend von einem biologischen Zustand induzierter künstlicher Zustand ist, gegebenenfalls im Gemisch mit Nichtkrebszellen, sowie daraus etablierte Zellinien. Bevorzugte Ausgestaltungen dieses Gegenstandes ergeben sich in Analogie zu den vorstehend beschriebenen Ausgestaltungen von erfindungsgemäßen Krebszellen mit biologischem Zustand.

Auch Zellbestandteile der erfindungsgemäßen Krebszellen, z.B. Lysate oder Fraktionen davon, zeichnen sich in ihrer Struktur und/oder Zusammensetzung dahingehend aus, daß sie sich von den erfindungsgemäßen Zellbestandteilen ableiten.

Gegenstand der vorliegenden Erfindung sind daher auch - ebenfalls unter den erfindungsgemäßen Begriff Krebszellmaterial zu fassende - Zellbestandteile, die aus den erfindungsgemäßen Krebszellen mit biologischem oder induziertem künstlichen Zustand abgeleitet sind. Diese Zellbestandteile können aus den entsprechenden Zellen in an sich bekannter Weise hergestellt werden.

Das erfindungsgemäße Krebszellmaterial kann in Form von Stoffbanken, z.B. Zellbanken oder Biorepositorien, angelegt sein. Eine zweckmäßige Aufbewahrung liegt im Bereich fachmännischen Könnens unter Berücksichtigung des aufzubewahrenden Materials. Für lebendes und/ oder empfindliches Material eignet sich insbesondere die Kryokonservierung, z.B. unter Stickstoff. In dieser Form kann das Krebszellmaterial zur gewerblichen Nutzung im Hinblick auf weitere Verwendungen hergerichtet sein. Zellbestandteile hingegen können unter Umständen als Chemikalie - je nach Empfindlichkeit auch bei höheren Temperaturen, gegebenenfalls unter Trockeneis- oder Eiskühlung oder sogar bei Raumtemperatur in zweckmäßiger Verpackung - gehandelt werden.

Ein weiterer Gegenstand der vorliegenden Erfindung sind Mittel, beispielsweise pharmazeutische oder tierarzneiliche Mittel zur diagnostischen und/oder therapeutischen Behandlung am Menschen oder an nicht-menschlichen tierischen Wesen, die - neben weiteren zweckmäßigen, fachmännisch auszuwählenden Komponenten, insbesondere Formulierungshilfen zur Verabfolgung, weiteren Wirkstoffen und/oder Komponenten eines diagnostischen Tests - erfindungsgemäßes Krebszellmaterial enthalten. Diese Mittel umfassen somit aus Körperflüssigkeiten isolierte Krebszellen, insbesondere disseminierte Tumorzellen, deren Zustand biologisch oder ein nach Isolierung induzierter künstlicher Zustand ist bzw. daraus abgeleitete Zellbestandteile. Anwendungen wie die Formulierung von autologen oder heterologen Impfstoffen im therapeutischen Bereich oder die Etablierung von Kontrollen in diagnostischen Testsystemen seien hier beispielhaft genannt.

Gegenstand der vorliegenden Erfindung ist auch die Verwendung des erfindungsgemäßen Krebszellmaterials als Therapeutikum, d.h. zur Herstellung pharmazeutischer und/oder tierarzneilicher Mittel für die Therapie. Auch als Target im diagnostischen, therapeutischen, tierexperimentellen oder wissenschaftlichen Bereich findet das erfindungsgemäße Krebszellmaterial Anwendung.

So kann das erfindungsgemäße Krebszellmaterial dazu verwendet werden, bestimmte Muster, insbesondere genomische Dispositionen und/oder Expressionsprofile, zu charakterisieren. In Abhängigkeit vom zugrundeliegenden Krankheitsbild, insbesondere der Art und des Verlauf einer Krebserkrankung gegebenenfalls unter Berücksichtigung bereits vorgenommener therapeutischer Maßnahmen, lassen sich für diagnostische und/oder therapeutische Verfahren brauchbare auf einen bestimmten Erfolg abzielende Handlungsanweisungen zur Verfügung stellen.

Die Verwendung als Therapeutikum bezieht sich insbesondere auf vitale Krebszellen und ganz besonders auf die Etablierung von Vakzinen. Neben der ebenfalls möglichen heterologen Immunisierung betrifft dies insbesondere die autologe Immunisierung. Zu diesem Zweck wird das erfindungsgemäße Krebszellmaterial gegebenenfalls in geeigneter Weise aufbereitet, so daß gegen die Oberfläche der Zellen oder gegen Zellbestandteile, nach Injektion in Form einer verträglichen Formulierung, Antikörper und/oder immunreaktive Zellen gebildet werden. Zu nennen ist auch die Verwendung als Drug-Vehicle, d.h. als spezifisches in-vivo-Transportmedium insbesondere für therapeutisch wirksame Substanzen. Im Rahmen einer weiteren therapeutischen Verwendung wird das entnommene Krebszellmaterial zunächst modifiziert - zu nennen wären hier beispielsweise Methoden, die unter den Schlagwörtern Mikroinjektion, Gentransfer, Antisense, knock out etc. bekannt sind - und dann dem zu behandelnden Individuum wieder zugeführt, beispielsweise durch Reinfusion.

Als Target findet das erfindungsgemäße Krebszellmaterial Verwendung im diagnostischen, therapeutischen, tierexperimentellen und wissenschaftlichen Bereich.

Die Verwendung im diagnostischen Bereich betrifft insbesondere die Charakterisierung von Krebszellen aus Körperflüssigkeiten. Die Charakterisierung beinhaltet sowohl die Identifizierung und den Nachweis der Krebszellen als solche, als auch die Bestimmung einer oder mehrerer Parameter an diesen Krebszellen. Im Hinblick auf menschliche oder nicht-menschliche tierische Individuen bezieht sich diese Verwendung insbesondere auf das in der WO 99/10528 beschriebene Verfahren zur Charakterisierung disseminierter und mikrometastasierter Krebszellen anhand von DNA und/oder RNA.

Gegenstand der vorliegenden Erfindung ist daher auch ein Verfahren zur Charakterisierung disseminierter und mikrometastasierter bzw. metastasierender Krebszellen anhand von DNA und/oder RNA, wobei man aus Körperflüssigkeit eines Individuums nach dem erfindungsgemäßen Verfahren abgetrennte Krebszellen anhand von DNA und/oder mRNA auf wenigstens ein krebsspezifisches Gen untersucht und die gleiche Untersuchung mit Nichtkrebszellen desselben Individuums zum Vergleich durchführt und gegebenenfalls auch aus Körperflüssigkeit eines Individuums auf herkömmliche Art und Weise gewonnene Zellen, in der Regel zellhaltige Fraktionen der betreffenden Körperflüssigkeit, anhand von mRNA auf wenigstens ein krebsspezifisches Gen untersucht. Besondere Ausgestaltungen und Ausführungsformen dieses Verfahrens ergeben sich unter Bezugnahme auf die in der WO 99/10528 in den Ansprüchen 1 bis 10 offenbarten Verfahren, insbesondere unter Berücksichtigung der im Glossar genannten Gene und weiterer in der WO 99/10528 offenbarter Kombinationen bestimmter Gene zur Vornahme von Multiparameter-Expressionsanalysen sowie genomischer Untersuchungen auf Onkogene und/oder mutierte Tumorsuppressorgene.

Unabhängig von und auch zusätzlich zur Charakterisierung anhand von DNA und/oder RNA kann man am erfindungsgemäßen Krebszellmaterial auch Proteine, Zucker, Glykosylierungsstrukturen, Ribozyme und ähnliches untersuchen, um disseminierte und mikrometastasierte bzw. metastasierende Krebszellen zu charakterisieren.

Insbesondere beinhaltet die Identifizierung und Charakterisierung isolierter Krebszellen die Durchführung krebsnachweisender Analysen, beispielsweise Analysen der Nukleinsäuren auf Mutationen, Insertionen, Deletionen, LOH, Amplifikationen, Aberrationen im Chromosomensatz und dergleichen; tumorbiologisch relevanter Analysen zur Messung verschiedenster zellphysiologischer Parameter, die beispielsweise mit der Metastasierung, dem Zellzyklus, der Proliferation oder der Apoptose von Krebszellen zusammenhängen; Analysen pharmakologisch relevanter Parameter, wobei die isolierten Zellen in vitro unter verschiedenen Bedingungen, beispielsweise unter Zusatz von Cytostatika, Antagonisten und dergleichen kultiviert werden, so daß verschiedene Therapieformen in vitro getestet und für jeden Patienten individuell optimiert werden können; Bioassays, in denen Aktivierungen, Inhibitionen oder sonstige Veränderungen der isolierten Krebszellen durch zellwirksame Moleküle, wie Cytokine, Chemokine, Hormone, Wachstumsfaktoren, Liganden, chemische oder biologische Analoga, die Apoptisierbarkeit der isolierten Krebszellen oder deren apoptisches Potential gegenüber anderen Targetzellen oder auch die Strahlungssensitivität der isolierten Krebszellen zur individuellen Dosisabschätzung für einen Patienten bestimmt werden können; cytologische Analysen, unter Anwendung bekannter Methoden, wie der Immunhistochemie, Gegenfärbung, FISH- oder sonstiger cytologischer Nachweis- und Färbeverfahren. Folgende Analysen seien beispielhaft genannt:
-- Krebsnachweisende DNA/RNA-Analysen, die Onkogene bzw. Tumorsupressorgene, wie p53, Gene der ras-Familie, erb-B2, c-myc, mdm2, c-fos, DPC4, FAP, nm23, RET, WT1 u.ä., LOH's, beispielsweise im Hinblick auf p53, DCC, APC, Rb u.ä. sowie BRCA1 und BRCA2 bei hereditären Tumoren, Mikrosatelliten-Instabilität von MSH2, MLH1, WT1 u.ä., tumoröse RNA's, wie CEA, Cytokeratine, z.B. CK20, MUC1, MAGE3, Muc18, Tyrosinase, PSA, PSM, BA46, Mage-1 u.ä., oder morphogene RNA's, wie Maspin, HCG, GIP, Motilin, hTG, SCCA-1, AR, ÖR, PR, verschiedene Hormone u.ä., betreffen;
-- Analysen tumorbiologisch-relevanter RNA's und Proteine, die das Metastasierungsprofil, d.h. die Expression von Angiogenese-, Motilitäts-, Adhäsions-und Matrixdegradations-molekülen, wie bFGF, bFGF-R, VEGF, VEGF-R's, wie VEGF-R1 oder VEGF-R2, E-Cadherin, Integrine, Selectine, MMP's, TIMP's, SF, SF-R u.ä., das Zellzyklus- bzw. Proliferationsprofil, wie Cycline (z.B. das Expressionsverhältnis von Cyclin D, E und B), Ki67, P120, p21, PCNA u.ä., oder das Apoptose-Profil, wie FAS (L+R), TNF (L+R), Perforin, Granzyme B, BAX, bcl-2, Caspase 3 u.ä., betreffen.

Die Durchführung derartiger Verfahren im Rahmen von Screening- Verfahren u.a. zur Tumorfrüherkennung und/ oder Lokalisation von Tumoren bietet sich an. Weiterhin sind spezifische Anwendungen zu nennen, beispielsweise der Nachweis eines bestimmten Karzinoms oder einer bestimmten Mutation, letzteres z.B. in der Nachsorge, z.B. als Verlaufskontrolle, insbesondere im Hinblick auf eine Mutation, auf deren Basis sich der Tumor entwickelt hat. Diese spezifischen Anwendungen können vorteilhafterweise als Testsysteme bereitgestellt werden, gegebenenfalls mit Anleitung und weiteren Testkomponenten, wie Primern, Kontrollen etc., beispielsweise in Form von Kits.

Weitere Verwendungen im diagnostischen Bereich betreffen die Qualitätskontrolle zellhaltiger Präparate, z.B. den Nachweis von Tumorzellen in Blutkonserven, in der Transplantationsmedizin zur Kontrolle von Stammzellpräparaten und sonstigen Transplantaten, gegebenenfalls nach Entfernung von Krebszellen, sowohl in autologen Präparaten als auch bei heterologen Präparaten, die unter Umständen auch dann Krebszellen enthalten können, wenn der Nachweis einer Erkrankung durch einen soliden Tumor noch nicht erfolgt ist. Im forensischen Bereich gelingt so die Verursacheridentitikation bei Tumorinfektionen, die durch Blutkonserven oder andere Transplantationsprodukte vermittelt werden.

Die Verwendung des erfindungsgemäßen Krebszellmaterials in der Therapie bezieht sich insbesondere auf die Therapieentwicklung, die Therapieauswahl, das Therapie-Monitoring und die Beurteilung evtl. bestehender Therapieresistenzen.

Im Rahmen der Therapieentwicklung ist beispielsweise das Drug-Targeting zu nennen, insbesondere der Einsatz des erfindungsgemäßen Krebszellmaterials bei der Identifizierung neuer therapeutischer Targets, der Identifizierung einer weiteren Zielgruppe für gegebenenfalls bereits zugelassene Medikamente, wobei man insbesondere die Expression des Targets und/oder Polymorphismen unter dem Einfluß bestimmter Therapeutika, wie z.B. Antikörpern, Liganden und Rezeptoren, Enzymen, Inhibitoren, Chemotherapeutika, Lektinen, Lipiden, katalytischen Substanzen etc. beurteilt, die Effektoren, wie zum Beispiel Signaltransduktionsfaktoren, oder Effekte, wie z.B. Präapoptose, Apoptose, Anergie, weitere den Zellzyklus betreffende Effekte etc. bestimmt, oder beispielsweise auf Veränderungen durch clonale Selektion ggf. vor und/oder nach Behandlung mit bestimmten Wirkstoffen untersucht. Weiterhin betrifft die Verwendung des erfindungsgemäßen Krebszellmaterials im Rahmen der Therapieentwicklung auch die Überprüfung therapeutischer Targets durch ex-vivo Untersuchungen, beispielsweise in Zellkulturen und an Tiermodellen. Im Rahmen der Wirkstoffentwicklung findet das erfindungsgemäße Krebszellmaterial Anwendung im Screening von Wirkstoffen, z.B. zur Identifizierung und Charakterisierung von Leitsubstanzen, z.B. Vektoren, Antisense-Molekülen, Ribozymen, Toxinen, Chemotherapeutika, Antihormonen, etc. In der Regel und vor allem nach einem High Throughput Screening (HTS) fallen im Rahmen des Drug Discovery Prozesses mehrere Hits oder Leitsubstanzen an, und eine weitere Einengung der weiter zu verfolgenden Substanzen kann ex-vivo, in-vitro und/oder in-vivo, z.B. im Tiermodell, an dem erfindungsgemäßen Krebszellmaterial vorgenommen werden. Dabei kann ein Bezug zur aktuellen Tumorsituation hergestellt werden.

In diesem Zusammenhang ist insbesondere die Entwicklung von Wirkstoffen gegen die Oberflächenstrukturen von Krebszellen zu erwähnen. Dem Fachmann sind Verfahren bekannt, mit denen Makromoleküle geschaffen werden können, die an bstimmte Strukturen binden, wobei Spezifität und Affinität durch selbst-optimierende Prozesse stetig verbessert werden. Unerwünschte Bindungseigenschaften werden durch Ausleseprozesse eliminiert. Unter Verwendung des erfindungsgemäßen Krebszellmaterials als Träger der gewünschten Strukturen lassen sich so spezifische Therapeutika entwickeln, wie Antikörper, Aptamere etc.

Auch im Sekundär-Screening finden die erfindungsgemäßen Krebszellen Anwendung. In der Regel müssen die im Primär-Screening identifizierten Substanzen noch optimiert werden, beispielsweise im Hinblick auf ihre Herstellbarkeit, die Synthesekosten, die Stabilität, kinetische Eigenschaften, ihr Metabolisierungsverhalten u.ä. Ein wichtiger Faktor ist die optimale Wirkung auf das therapeutische Target. Diese und weitere Optimierungsprozesse können in vorteilhafter Weise durch ex-vivo Untersuchungen unter Verwendung der erfindungsgemäßen Tumorzellen vorgenommen werden, da diese eines der wesentlichen therapeutischen Targets darstellen. Durch die Verwendung dieser Krebszellen kann bei der Wirkstoffentwicklung auch der krebszellspezifische Metabolismus berücksichtigt werden, was insbesondere für die Entwicklung von Prodrugs von erheblicher Bedeutung ist. Wünschenswert ist eine spezifische Aktivierung in oder an der Zielzelle, beispielsweise unter Abspaltung anderer Bestandteile aus Wirkstoffkonjugaten, die auf diese Weise erst durch eine krebszellspezifische Aktivität in eine wirksame Form überführt werden. Ein weiteres Einsatzgebiet der erfindungsgemäßen Krebszellen ist die Entwicklung und/oder Überprüfung von Vektoren, beispielsweise für.gentherapeutische Ansätze, mit denen Nukleinsäure effektiv in die Krebszellen eingeschleust werden können. Unter Verwendung des erfindungsgemäßen Krebszellmaterials können Vektoren an die Struktur der Zielzelle, insbesondere an ihre genetische Ausstattung, angepaßt werden, und es kann ex-vivo die Effektivität des Einschleusungsprozesses überprüft werden.

Weiterhin findet das erfindungsgemäße Krebszellmaterial Verwendung bei der Bestimmung der therapeutischen Breite eines Wirkstoffs, indem man in-vitro die Wirkung eines Wirkstoffes auf Krebszellen und Nichtkrebszellen miteinander vergleicht. Beispielsweise soll sich eine zytostatische Therapie im Idealfall ausschließlich auf die Krebszellen auswirken. Mit dieser Anwendung lassen sich eventuelle Nebenwirkungen erkennen.

Mit Blick auf die vorstehenden Ausführungen ergibt sich unmittelbar, daß das erfindungsgemäße Krebszellmaterial auch dazu verwendet werden kann, eine im Einzelfall geeignete Therapie auszuwählen, die nicht nur durch die Art des Krebses bestimmt wird, sondern vom jeweiligen Individuum und vom Stadium der Erkrankung abhängt.

Verwendung finden die erfindungsgemäßen Krebszellen auch im Therapiemonitoring, d.h. der zeitabhängigen Beurteilung einer therapeutischen Maßnahme. Diese Anwendung ergibt sich sowohl bei einem bestehenden soliden Tumor, der entweder diagnostiziert oder, in einem Tier, auch transplantiert oder induziert worden sein kann. Möglich ist auch die Übertragung erfindungsgemäßen Krebszellmaterials auf ein Tier, wie Mäusen, Ratten und anderen Säugern, Hühnereiern und ähnlichem. Es können Kinetiken zum zeitlichen Verlauf der Krebszell-Konzentration in der untersuchten Körperflüssigkeit und der Entwicklung ausgewählter Parameter der Krebszellen aufgenommen werden.

Weiterhin können unter Verwendung des erfindungsgemäßen Krebszellmaterials ggf. vorhandene Therapieresistenzen, beispielsweise gegen Chemotherapeutika und andere Wirkstoffe gegen Krebs erkannt werden. Hier bieten sich sowohl in-vitro als auch ex-vivo Analysemethoden und Testsysteme an.

Auch kann das erfindungsgemäße Krebszellmaterial zur Etablierung hochspezifischer Tumormodelle verwendet werden, z.B. um gezielt Tumore zu induzieren oder die Auswirkungen von erfindungsgemäßem Krebszellmaterial auf und dessen Verhalten in Organismen zu beurteilen. Dazu können die Krebszellen, gegebenenfalls in markierter Form, geeigneten Versuchstieren, beispielsweise durch Reinfusion, zugeführt werden.

Weiterhin wird das erfindungsgemäße Krebszellmaterial zur Untersuchung zahlreicher wissenschaftlicher und praktischer Fragestellungen verwendet, beispielsweise zur Identifizierung und/oder Charakterisierung von Tumorinduktoren und Tumorenhancern, wie z.B. Viren, Bakterien, intrazellulären Parasiten, alkylierenden und anderen mutagenen Substanzen, etc.; zur Bereitstellung neuer Strukturen, beispielsweise zur Identifizierung und Isolierung neuer Gene, Genprodukte, Proteine, Glykosilierungsstrukturen, etc., u.a. im Hinblick auf neue therapeutische Targets und/oder Diagnose-Tools; zur Induktion von Knock outs und zu Funktionsuntersuchungen; zur Identifizierung bestimmter Expressionsprofile, beispielsweise veränderter Genexpressionsmuster in Abhängigkeit vom tumorbiologischen Zustand einer Krebszelle, u.a. im Hinblick auf die Metastasierung, Resistenzentwicklung vor, während und/oder nach Therapie, oder im Vergleich zu Zellen aus Normal-, Primärtumor-, Rezidivtumor- oder aus Metastasengewebe; zur Identifizierung bestimmter Polymorphismen oder Kombinationen; zur Untersuchung der Variabilität genetischer Information, beispielsweise zur Identifizierung struktureller Nukleinsäure-Änderungen, wie Mutationen, Splice-Varianten, etc.; im Proteomics-Bereich, sowohl analytisch als auch präparativ ggf. mit Folgeanwendungen, beispielsweise mit Hilfe konfokaler Laserscanning-Mikroskopie oder anderen Verfahren, wie Maldi-Tof, ES-MS/MS; zur Untersuchung von Zell/Zell-Interaktionen, wozu man beispielsweise erfindungsgemäßes Krebszellmaterial zusammen mit manipulierten Killerzellen inkubieren kann; zur Isolierung und Charakterisierung von Krebszellbestandteilen sowohl aus kultivierten als auch aus nicht-kultivierten Krebszellen, beispielsweise Proteine, wie Lipo-, Glycoproteine, etc., Peptide, Lipide, Kohlenhydrate, etc. An dem erfindungsgemäßen Krebszellmaterial lassen sich Zusammenhänge von Entstehung, Entwicklung und Wirkung therapeutischer Ansätzen aufklären, und darauf aufbauend werden neue therapeutische Ansätze zur Verfügung gestellt, vor allem auch im Hinblick auf die Rationale für Kombinationen therapeutischer Ansätze.

Erfordert die Identifizierung und Charakterisierung der isolierten Krebszellen eine vorherige Isolierung von Nukleinsäuren, Proteinen oder anderen Zellbestandteilen, so sind dem Fachmann eine Vielzahl verschiedener Verfahren bekannt, mit denen er dieses bewerkstelligen kann. Beispielhaft seien hier lediglich einige wenige Methoden aufgezeigt.

Zur Isolierung genomischer DNA kann man die Zellen, beispielsweise unter Einwirkung von Detergentien und/oder Proteinasen, lysieren, Proteine entfernen und die DNA, beispielsweise durch Ausfällen mit bekannten organischen Lösungsmitteln, isolieren. Verfahren auf Basis von Guanidinisothiocyanat und Phenol enthaltenden Lösungen werden bevorzugt. Auch eine chromatographische Aufreinigung, d.h. eine Trennung von Nukleinsäuren und anderen Zellbestandteilen und/oder eine Trennung von Nukleinsäuren unterschiedlicher Art, beispielsweise mittels Extraktion an festen Phasen, wie Silikaten und ähnlichem, z.B. mit handelsüblichen Spin-Säulen, kann sinnvoll sein. Weitere bekannte Methoden, wie Sondentechniken, Elekrophorese, Elektroosmose, osmotischer Schock, können zweckmäßig sein. Ähnliche Verfahrensmaßnahmen finden bei der Isolierung von Gesamt-RNA Anwendung. Aus dieser Gesamt-RNA kann wiederum mRNA isoliert werden, indem man z.B. auf Oligo-(dT) basierende Systeme verwendet. Die Wahl eines zweckmäßigen Protokolls unterliegt fachmännischem Wissen.

Die isolierten Nukleinsäuren können dann zur weiteren Identifizierung und Charakterisierung in einer Vielzahl von Applikationen eingesetzt werden. Hierzu gehören beispielsweise die PCR, RT-PCR, DD-RT-PCR, cDNA-Synthese, Prirnerextension, restriktionsenzymatische Verdauung, Southern-Blotting, Markierungs- und Modifizierungsreaktionen, Northern-Blotting, Klonierung, Sequenzierung, in vitro-Transcription oder in vitro-Translation. Einige der vorstehend genannten Applikationen können auch an einer oder wenigen Zellen ohne vorherige Isolierung von Nukleinsäuren vorgenommen werden, insbesondere die RT-PCR. Die Wahl einer geeigneten Applikation hängt nicht nur von der Art der Nukleinsäure, sondern auch von der genetischen Information ab, anhand derer die Krebszellen identifiziert und charakterisiert werden sollen.

Besonders vorteilhaft ist die erfindungsgemäß resultierende hohe Reinheit und isolationstechnische Unberührtheit der isolierten Krebszellen. Dies ermöglicht es, bestimmte Funktionstests z.B. im Hinblick auf Pharmakogenomics (Test mit bestimmten Wirkstoffen) oder auf Veränderungen der isolierten Tumorzellen (Gen-Therapy, Gen-Replacement) durchzuführen. Des weiteren kann, durch die Reinheit der Zellen bedingt, ein sogenanntes "Drug-Targeting" durchgeführt werden. So ist z.B. aufgrund einer nachgewiesenen erb-B2 Amplifikation eine Therapie mit anti-erb-B2 Antikörpern oder anderen Liganden angezeigt; Progesteron- oder Östrogenrezeptor-exprimierende Tumorzellen sind einer sogenannten anti-Hormon-Therapie zugänglich. Weist man in den Krebszellen beispielweise eine Mutation des Genes für β-Tubulin nach, so würde dies eine Kontraindikation von Taxol® darstellen. Gleiches gilt für den Nachweis bestimmter Splicevarianten des Östrogenrezeptors im Hinblick auf Tamoxifen.

Des weiteren betrifft die vorliegende Erfindung Sets zur Isolierung und gegebenenfalls zur anschließenden Identifizierung und Charakterisierung disseminierter und metastasierter Krebszellen. Auch Sets zur Depletion von Krebszellen aus zellhaltigen Präparaten, insbesondere Körperflüssigkeiten oder Isolaten sind Gegenstand der Erfindung. Derartige Sets sollten möglichst einfach zu handhaben und im wesentlichen gebrauchsfertig sein. Eine bevorzugte Anordnung bietet die Sets in Kit-Form an. Essentielle Komponente geeigneter Sets ist wenigstens ein erfindungsgemäßes Sieb. Dieses Sieb kann im Sinne von ready-to-use bereits der Verwendung entsprechend angepaßt sein, es kann aber auch als eine Art Rohmaterial dem Set beigefügt sein, das vom Benutzer dann den jeweiligen Anforderungen entsprechend angepaßt, beispielsweise auf bestimmte Labware zugeschnitten werden kann. Zusätzlich können Komponenten vorhanden sein, die es ermöglichen,
- zellhaltige Körperflüssigkeit oder Teile davon durch das Sieb zu führen, beispielsweise säulenartige Teile, in denen das Sieb zweckmäßig angeordnet und vorzugsweise auch wieder entfernt werden kann;
- den Siebdurchlauf aufzufangen;
- die Zellen oder Zellbestandteile vom Sieb abzulösen und/ oder diese dann aufzunehmen, beispielsweise Lösungen, wie Puffer, Kulturmedien oder organische Lösungsmittel und/oder Lösungsgemische, wie Ethanol, Chloroform, Isoamylalkohol, Isopropanol, Guanidinisothiocyanat, Phenol und Gemische davon, z.B. Trizol®, vorzugsweise als gebrauchsfertige Lösungen oder Lösungsmittelgemische, die in vorzugsweise zentrifugierbaren Behältnissen und auch getrennt von diesen angeboten werden können;
- Nukleinsäuren, Proteine oder andere Zellbestandteile der isolierten Krebszellen zu isolieren oder zumindest für eine im Hinblick auf anschließende Analysen zweckmäßige Isolierung vorzubereiten, beispielsweise die zuvor genannten Lösungen, Spin-Säulen mit geeigneten Festphasen, Oligo-dT-Systeme u.ä. Derartige Sets sind universell einsetzbar und weitgehend von der Art der gegebenefalls durchzuführenden vorbereitenden Aufarbeitung der zellhaltigen Körperflüssigkeit und den sich anschließenden Verwendungen, z.B. den zur Identifizierung und Charakterisierung isolierter Krebszellen vorzunehmenden Analysen, unabhängig.
   Ferner können Komponenten vorhanden sein, die es ermöglichen,
- eine vorbereitende Aufarbeitung der zellhaltigen Körperflüssigkeit durchzuführen, beispielsweise zur Isolierung von Zellen oder bestimmter zellhaltiger Fraktionen aus dieser Körperflüssigkeit;
- die zur Identifizierung und Charakterisierung isolierter Krebszellen angestrebten Analysen, insbesondere Untersuchungen der oben genannten Gene und Proteine, durchzuführen, beispielsweise Primer, Mittel zur Amplifikation, Detektion und/oder Kontrollen. Bei den Kontrollen kann es sich auch um erfindungsgemäßes Krebszellmaterial handeln.
Aufgrund der Vielfalt derartiger Maßnahmen sind diese Komponenten - wenn überhaupt - in der Regel nur in beschränktem Umfang in dem erfindungsgemäßen Set untergebracht, d.h. es wird jeweils auf eine bestimmte Körperflüssigkeit und/oder eine oder wenige Analysen abgestellt. Es handelt sich dann um Kits zur Durchführung einer Identifizierung und Charakterisierung isolierter Krebszellen anhand eines oder weniger Parameter.

Die nachfolgenden Beispiele sollen die Erfindung näher erläutern, ohne sie zu beschränken.

### Beispiel 1

### Isolierung disseminierter Krebszellen aus Blut.

10 ml heparinisiertes Blut werden abzentrifugiert (400 g; 10 min; RT). Das überstehende Plasma wird abgenommen. Die pelletierten Zellen werden in 12 ml PBS aufgenommen. Nach Dichtegradientenzentrifugation (Nycodenz 1.077; 800 g; 30 min, RT) werden die Interphasezellen (mononukleäre Zellen, kurz MNC) abgenommen und 2 x in 10 ml PBS (1 mM EDTA) gewaschen (400 g; 10 min; 40C). Die MNC's werden in 10 ml PBS aufgenommen (1 mM EDTA, 0,5 % BSA). Als Bezugsgröße wird 1 ml dieses Zellgemisches abgenommen (Kontrollfraktion). Die restlichen 9 ml Zellgemisch werden über eine Säule durch ein aus PE-Fäden gewebtes Sieb mit 20 µm Maschenweite (vertrieben von der SEFAR AG, Rüschlikon, Schweiz) geführt und der Siebdurchlauf gesammelt. Die Säule wird 5 x mit je 10 ml PBS (1 mM EDTA) gewaschen. Das Sieb wird herausgenommen, umgedreht und in einem Reaktionsgefäß mit 0,7 ml Trizol^{®} inkubiert (5 min; RT). Das Sieb wird im Reaktionsgefäß oberhalb der Trizol^{®}-Lösung plaziert und abzentrifugiert (200 g; 30 s; RT). Das trockene Sieb wird entfernt und die Trizol^{®}-Lösung der weiteren RNA/DNA-Isolation zugeführt.

Alternativ zur Inkubation des Siebes in Trizol^{®} kann das Sieb der Säule entnommen, umgedreht und in PBS (1 mM EDTA, 0,5 % BSA) überführt werden, und die Zellen können durch Zentrifugation (400 g; 10 min, 4°C) pelletiert werden.

### Beispiel 2

Isolierung von CD45-positiven Zellen (Kontrollfraktion) Zur Isolierung von CD45-positiven Lymphozyten als Kontrollfraktionen und auch für die Messung von LOH's werden jeweils 1/10 der MNC's vor und nach dem Siebvorgang (siehe Beispiel 1) abgenommen. Diese werden in ein 1 ml PBS (0.5% BSA, 100 µg hu-IgG) enthaltendes Reaktionsgefäß überführt. Dazu werden 50 µl gewaschene anti-CD45 Microbeads gegeben. Der Ansatz rotiert bei 4°C 20 min. Anschließend wird das Reaktionsgefäß so an einer Magnetleiste positioniert, daß die Microbeads (gebunden an CD45-positive MNC's) an der Gefäßwand pelletieren. Durch dreimaliges Waschen der Bead-Zell-Aggregate erhält man eine reine Population CD45-positiver Lymphozyten, welche dann in Trizol^{®} gelöst der Isolation von Nukleinsäuren zugeführt werden können. CD45-Isolate der MNC's vor dem Siebvorgang werden als Kontrollfraktion A bezeichnet, CD45-positive Isolate der MNC's nach dem Siebvorgang als Kontrollfraktion B.

### Beispiel 3

### DNA-Analysen

Genomische DNA wird in herkömmlicher Weise aus den in den Beispielen 1 und 2 erhaltenen Trizol^{®}-Lösungen isoliert. Die DNA wird dann durch PCR amplifiziert, wobei man die nachstehend angegebenen Primer und Parameter verwendet.

### 1. Analyse der p53, Rb, DCC und APC Allele:

Pro PCR-Ansatz werden folgende Reagenzien zusammengegeben (µl):

| | |
|---|---|
| 10-fach PCR-Puffer | 5 |
| 20 mM dNTP | 0.5 |
| Primer A | 0.5 |
| Primer B | 0.5 |

| TAQ-Polymerase + TAQ-Start-Antikörper | |
|---|---|
| 1:1 | 0.5 |
| H₂O | 40 |
| DNA | 3 |

Folgende Temperaturprofile werden benutzt:

| für APC, Rb und DCC: | |
|---|---|
| 95 °C | 5 min |
| 94 °C | 30 sec |
| 53 °C | 30 sec 35 x |
| 72 °C | 30 sec |
| 72°C | 5 min |

| für p53 | |
|---|---|
| 95 °C | 5 min |
| 94 °C | 30 sec |
| 62 °C | 30 sec 35 x |
| 72 °C | 30 sec |
| 72°C | 5 min |

Als Primerpaare werden benutzt:
p53-LOH:
   A : 5'-Fl-Agg gAT ACT ATT CAg CCC CAg gTg
   B : 5'-ACT gCC ACT CCT TgC CCC ATT C
APC-LOH
   A : 5'-FAM gTA AgC Agg ACA AgA TgA Cag
   B : 5'-gCT ATT CTC TCA ggA TCT Tg
DCC-LOH
   A : 5'-HEX-gAT gAC ATT TTC CCT CTA g
   B : 5'-gTg gTT ATT gCC Ttg AAA Ag
Rb-LOH
   A : 5'-FAM-CTC CTC CCT ACT TAC Ttg T
   B : 5'-AAT TAA CAA ggT gTg gTg g

Alle Primer werden in einer Konzentration von 20 pmol/µl gelagert. Normale DNA wird immer als negative Kontrolle eingesetzt. Alle PCR-Amplifikate der LOH- und Amplifikationsanalysen werden auf einem ABI-Prism Genescan Genetic Analyser gemessen und ausgewertet.

### 2. Amplifikationsanalyse von erb-B2 und c-myc

Gemessen wird eine Coamplifikation von erb-B2 (c-myc) versus β-Globin.

Pro PCR-Ansatz werden folgende Reagenzien zusammengegeben (µl):

| | c-myc | erb-B2 |
|---|---|---|
| 10-fach PCR-Puffer | 5 | 5 |
| MgCl₂ (25 mM) | 4 | 4 |
| 20 mM dNTP | 0.25 | 0.25 |
| Primer A | 2 | 1.5 |
| Primer B | 2 | 1.5 |
| β-Globin-Primer A | 0.2 | 0.5 |
| β-Globin-Primer B | 0.2 | 0.5 |
| (NH₄)₂SO₄ | 7.5 | 7.5 |
| AmpliTaq Gold | 0.4 | 0.4 |
| H₂O | 25.45 | 25.85 |
| DNA | 3 | 3 |

Folgendes Temperaturprofil wird benutzt:

| | | |
|---|---|---|
| 95 °C | 10 min | |
| 95 °C | 60 sec | |
| 60 °C | 60 sec | 32x |
| 72 °C | 60 sec | |
| 72°C | 3 min | |

Als Primerpaare werden benutzt:
erb-B2
   A : 5'-HEX-Cgg ATC TTC TgC TgC CgT Cg
   B : 5'-CCT Ctg Acg TCC ATC ATC TC
c-myc
   A : 5'-HEX-CgT ATT CAT gCC Ttg TAT Ttg
   B : 5'-CTT CTT CAT CTT CTT gTT CC
Globin
   A : 5'-FAM-ACA CAA Ctg TgT TCA CTA gC
   B : 5'-CAA CTT CAT CCA CgT TCA CC

Alle Primer werden in einer Konzentration von 20 pmol/µl gelagert. Normale DNA wird immer als negative Kontrolle und 5-fach amplifizierte (erb-B2/c-myc) DNA als positive Kontrolle eingesetzt. Alle PCR-Amplifikate der LOH- und Amplifikationsanalysen werden auf einem ABI-Prism Genescan Genetic Analyser gemessen und ausgewertet.

### Beispiel 4

### Klinische Anwendung

14 Patienten mit unterschiedlichen Karzinomen, ein Patient mit malignem Melanom, sechs Kontrollspender und ein im Vorbefund auffälliger "Normalspender" wurden untersucht. Es wurden Analysen tumorspezifischer und tumorassoziierter RNA's sowie genomische Analysen auf "allele imbalance" (LOH) der Gene p53, Rb, APC und DCC und Amplifiktionsanalysen der Onkogene c-myc und erb-B2 durchgeführt.

Gemäß Beispiel 1 wurden aus dem Blut eines jeden Patienten die Kontrollfraktion, der Siebdurchlauf und der Siebrückstand erhalten. Gemäß Beispiel 2 wurden aus der Kontrolllraktion und dem Siebdurchlauf CD45-positive Zellen als "Wildtyp-Kontrolle" isoliert (Fraktionen A und B). Die vorstehend genannten Analysen wurden an den CD45-positiven Zellen der Kontrollfraktion (A; Bezugsgröße), den CD45-positiven Zellen des Siebdurchlaufs (Fraktion B) und den Zellen des Siebrückstandes (Fraktion C) durchgeführt.

Für Patienten und Kontrollspender wurden die Ergebnisse der genomischen Analysen als relative Allel-Unterschiede bezogen auf die CD45-Kontrollfraktion A (Bezugsgröße) angegeben. Als Cut-off-Wert der LOH-Analysen wurde, bezogen auf die Kontrolle, ein Wert von ≤ 0,5, d.h. mindestens 50 % Unterschied, als positiv gesetzt. Bei den Amplifikationsanalysen wurde ein Cut-off-Wert von 2,0 gesetzt.

Für fünf der 15 untersuchten Patienten mit etabliertem Karzinom und Melanom konnte nachgewiesen werden, daß disseminierte Krebszellen aus peripherem Blut mit dem erfindungsgemäßen Verfahren isoliert werden konnten. Dies gelang auch bei einem der Kontrollspender, dessen Vorbefund (Expression der CEA-, CK20- und MUC1-RNA's) Hinweise auf disseminierten Krebszellen gab.

### Beispiel 5

### Charakterisiserung des biologischen Zustandes disseminierter Tumorzellen

Einer 53-jährigen Patientin mit einem vor 16 Monaten diagnostizierten und behandelten (6xCMF, Radiatio, Tamoxifen) Mammakarzinom (pT2pN1M0, G3) wurde eine Blutprobe entnommen. Disseminierte Tumorzellen wurden gemäß Beispiel 2 isoliert. Diese wiesen ein LOH des p53-Gens, eine Mutation des p53-Gens und eine Amplifikation von c-erbB-2 auf. Die quantitative und auf GADPH normierte Untersuchung der Cyclinexpression ergab folgendes Resultat:
Cyklin D: 2,5
Cyclin E: 0
Cyclin B: 0

Die ausschließliche Expression von Cyclin D spricht für eine G0/1-Phase. Keine der isolierten Zellen befand sich in einem mitotischen Stadium. Die isolierten Tumorzellen waren nicht proliferativ.

Einem 64-jährigen Patienten mit einem vor 6 Monaten diagnostizierten und behandelten (5-FU) Colonkarzinom (Dukes C) wurde eine Blutprobe entnommen. Disseminierte Tumorzellen wurden gemäß Beispiel 2 isoliert. Diese wiesen ein LOH des DCC-Gens, ein LOH des E-Cadherin-Gens und eine Amplifikation von c-myc auf. Die quantitative und auf GADPH normierte Untersuchung der Cyclinexpression ergab folgendes Resultat:
Cyklin D: 0,7
Cyclin E: 4,3
Cyclin B: 10,3

Die Expression aller drei Cycline spricht für für einen proliferativen Zustand der isolierten Tumorzellen.

## Patentansprüche

1. Verfahren zur Isolierung von disseminierten Tumorzellen aus zellhaltigen Körperflüssigkeiten, **dadurch gekennzeichnet, daß** man die zellhaltige Körperflüssigkeit oder Teile davon durch ein Sieb mit einer Maschen- oder Porenweite von etwa 15 bis 30 µm oder ein Sieb mit einem absoluten oder nominalen Rückhaltevermögen von etwa 15 bis 30 µm führt und die zurückgehaltene Zellfraktion gewinnt.

2. Verfahren nach Anspruch 1, **dadurch gekennzeichnet, daß** das Sieb eine Maschen- oder Porenweite von etwa 20 µm oder ein absolutes oder nominales Rückhaltevermögen von etwa 20 µm aufweist.

3. Verfahren nach Anspruch 1 oder 2, **dadurch gekennzeichnet, daß** die auf dem Sieb zurückgehaltenen disseminierten Tumorzellen frei von einem Trennmittel, welches einen zu Isolationszwecken verwendeten Liganden umfaßt, sind.

4. Verfahren nach einem der Ansprüche 1 bis 3, **dadurch gekennzeichnet, daß** man zunächst eine zellhaltige Fraktion aus der Körperflüssigkeit isoliert und anschließend siebt.

5. Verfahren nach einem der Ansprüche 1 bis 4, **dadurch gekennzeichnet, daß** man die auf dem Sieb zurückgehaltenen disseminierten Tumorzellen, gegebenenfalls im Gemisch mit Nichtkrebszellen, vom Sieb ablöst.

6. Verfahren nach einem der Ansprüche 1 bis 5, **dadurch gekennzeichnet, daß** man eine Flüssigkeit in entgegengesetzter Richtung durch das Sieb führt.

7. Verfahren nach einem der vorhergehenden Ansprüche, **dadurch gekennzeichnet, daß** man als Körperflüssigkeit Blut, Knochenmark, Lymphe, Urin, Sputum, Fruchtwasser, Punktate, Colon-, Lungen-, Bronchiallavage, Blasenspülflüssigkeit, oder Fäces verwendet.

8. Verfahren nach Anspruch 7, **dadurch gekennzeichnet, daß** man als Körperflüssigkeit Blut oder Knochenmark verwendet.

9. Verfahren zur extrakorporalen Eliminierung von disseminierten Tumorzellen aus zellhaltigen Präparaten, **dadurch gekennzeichnet, daß** man das zellhaltige Präparat durch ein Sieb mit einer Maschen- oder Porenweite von etwa 15 bis 30 µm oder ein Sieb mit einem absoluten oder nominalen Rückhaltevermögen von etwa 15 bis 30 µm führt.

10. Verfahren zur Charakterisierung disseminierter Tumorzellen anhand von DNA und/oder RNA, wobei man aus Körperflüssigkeit eines Individuums disseminierte Tumorzellen nach einem Verfahren der Ansprüche 1 bis 8 abtrennt und anhand von DNA und/oder mRNA auf wenigstens ein krebsspezifisches Gen untersucht, und die gleiche Untersuchung mit Nichtkrebszellen desselben Individuums zum Vergleich durchführt.

11. Verwendung eines Siebes mit einer Maschen- oder Porenweite von etwa 15 bis 30 µm oder eines Siebes mit einem absoluten oder nominalen Rückhaltevermögen von etwa 15 bis 30 µm zur Isolierung disseminierter Tumorzellen aus zellhaltigen Körperflüssigkeiten.

12. Disseminierte Tumorzellen umfassendes Zellgemisch, **gekennzeichnet durch** einen polyklonalen Tumorzellanteil von wenigstens 50%, erhältlich **durch** ein Verfahren nach einem der Ansprüche 1 bis 8.

13. Zellgemisch nach Anspruch 12, **gekennzeichnet durch** einen polyklonalen Tumorzellanteil von wenigstens 90%.

14. Zellgemisch nach Anspruch 12 oder 13, **dadurch gekennzeichnet, daß** die Tumorzellen frei von herkömmlicherweise zu Isolationszwecken verwendeten Liganden sind.

15. Verfahren zur Isolierung von Zellbestandteilen disseminierter Tumorzellen, wobei man die Zellbestandteile aus wenigstens einem Zellgemisch der Ansprüche 12 bis 14 oder einer Fraktion davon in an sich bekannter Weise gewinnt.

16. Verfahren zur Etablierung von Zellinien disseminierter Tumorzellen, wobei man wenigstens ein Zellgemisch der Ansprüche 12 bis 14 oder eine Fraktion davon kultiviert.

17. Zellgemisch nach einem der Ansprüche 12 bis 14 zur Verwendung als Therapeutikum.

18. Pharmazeutisches oder tierarzneiliches Mittel, umfassend wenigstens ein Zellgemisch nach einem der Ansprüche 12 bis 14 und weitere Formulierungshilfen, Wirkstoffe und/oder Komponenten eines diagnostischen Tests.

## Claims

1. A method for isolating disseminated tumor cells from cell-containing body fluids, **characterized in that** the cell-containing body fluid or part thereof is passed trough a screen having a mesh or pore width of about 15 to 30 µm or a screen having an absolute or nominal retention capacity of about 15 to 30 µm and the retained cell fraction is obtained.

2. The method of claim 1, **characterized in that** the screen has a mesh or pore width of about 20 µm or an absolute or nominal retention capacity of about 20 µm.

3. Method of claim 1 or 2, **characterized in that** the disseminated tumor cells retained on the screen are free from a separating agent which comprises a ligand used for isolation purposes.

4. The method as claimed in any one of claims 1 to 3, **characterized in that** a cell-containing fraction is first isolated from the body fluid and subsequently screened.

5. The method of any one of claims 1 to 4, **characterized in that** the disseminated tumor cells retained on the screen are removed from the screen, optionally in a mixture with non-cancer cells.

6. The method of any one of claims 1 to 5, **characterized in that** a liquid is passed through the screen in the opposite direction.

7. The method of any one of the preceding claims, **characterized in that** the body fluid used is blood, bone marrow, lymph, urine, sputum, amniotic fluid, aspirates, colon lavage, lung lavage, bronchial lavage, bladder irrigation fluid, or faeces.

8. The method of claim 7, **characterized in that** the body fluid used is blood or bone marrow.

9. A method for extracorporal elimination of disseminated tumor cells from cell-containing preparations, **characterized in that** the cell-containing preparation is passed through a screen having a mesh of pore width of about 15 to 30 µm or an absolute or nominal retention capacity of about 15 to 30 µm.

10. A method for characterizing disseminated tumor cells on the basis of DNA and/or RNA, wherein disseminated tumor cells are removed from body fluid of an individual using a method of claims 1 to 8 and are tested on the basis of DNA and/or mRNA for at least one cancer-specific gene and the same test is performed on non-cancer cells or the same individual for comparison.

11. The use of a screen having a mesh or pore width of about 15 to 30 µm or an absolute or nominal retention capacity of about 15 to 30 µm for isolating disseminated tumor cells from cell-containing body fluids.

12. A cell mixture comprising disseminated tumor cells **characterized by** a polyclonal tumor cell content of at least 50 %, obtainable by a method of anyone of claims 1 to 8.

13. The cell mixture of claim 12, **characterized in that** by a polyclonal tumor cell content of at least 90 %.

14. The cell mixture of claim 12 or 13, **characterized in that** the tumor cells are free from ligands conventionally used for isolation purposes.

15. A method for isolating cell components of disseminated tumor cells, wherein the cell components are obtained from at least one cell mixture of claims 12 to 14 or a fraction thereof in a manner known per se.

16. A method for establishing cell lines of disseminated tumor cells, wherein at least one cell mixture of claims 12 to 14 or a fraction thereof is cultured.

17. A cell mixture of claims 12 to 14 for use as therapeutic agent.

18. Pharmaceutical or veterinary composition comprising at least one cell mixture of any one of claims 12 to 14 and further formulation excipients, active substances and/or components of a diagnostic test.

## Revendications

1. Procédé permettant d'isoler des cellules tumorales disséminées, à partir de fluides corporels contenant des cellules, **caractérisé en ce que** l'on fait passer un fluide corporel contenant des cellules ou une partie de ce fluide à travers un crible dont les mailles ou les pores sont larges d'à peu près 15 à 30 µm, ou à travers un crible présentant une capacité de retenue nominale ou absolue d'à peu près 15 à 30 µm, et l'on récupère la fraction cellulaire retenue.

2. Procédé conforme à la revendication 1, **caractérisé en ce que** le crible présente des mailles ou des pores larges d'à peu près 20 µm, ou une capacité de retenue nominale ou absolue d'à peu près 20 µm.

3. Procédé conforme à la revendication 1 ou 2, **caractérisé en ce que** les cellules tumorales disséminées retenues sur le crible sont exemptes d'un agent de séparation comprenant un ligand utilisé en vue de l'isolement.

4. Procédé conforme à l'une des revendications 1 à 3, **caractérisé en ce qu'**on isole d'abord, à partir du fluide corporel, une fraction contenant des cellules, que l'on passe ensuite au crible.

5. Procédé conforme à l'une des revendications 1 à 4, **caractérisé en ce qu'**on enlève du crible les cellules tumorales disséminées retenues sur le crible, éventuellement mêlées à des cellules non-cancéreuses.

6. Procédé conforme à l'une des revendications 1 à 5, **caractérisé en ce qu'**on fait passer un liquide en sens contraire à travers le crible.

7. Procédé conforme à l'une des revendications précédentes, **caractérisé en ce qu'**on utilise, en tant que fluide corporel, du sang, de la moelle osseuse, de la lymphe, de l'urine, des crachats, du liquide amniotique, un liquide ponctionné, des liquides de lavage du côlon, des poumons, des bronches ou de la vessie, ou des fèces.

8. Procédé conforme à la revendication 7, **caractérisé en ce que** l'on utilise, en tant que fluide corporel, du sang ou de la moelle osseuse.

9. Procédé permettant d'éliminer, hors du corps, des cellules tumorales disséminées de préparations contenant des cellules, **caractérisé en ce que** l'on fait passer une préparation contenant des cellules à travers un crible dont les mailles ou les pores sont larges d'à peu près 15 à 30 µm, ou à travers un crible présentant une capacité de retenue nominale ou absolue d'à peu près 15 à 30 µm.

10. Procédé de caractérisation, par un ADN et/ou un ARN, de cellules tumorales disséminées, dans lequel on sépare des cellules tumorales disséminées d'avec un liquide corporel d'un individu, en suivant un procédé conforme à l'une des revendications 1 à 8, puis on analyse un ADN et/ou un ARNm de ces cellules, pour en étudier au moins un gène spécifique de cancer, et l'on effectue, pour comparaison, la même analyse sur des cellules non-cancéreuses du même individu.

11. Emploi d'un crible dont les mailles ou les pores sont larges d'à peu près 15 à 30 µm, ou d'un crible présentant une capacité de retenue nominale ou absolue d'à peu près 15 à 30 µm, pour isoler des cellules tumorales disséminées, à partir de fluides corporels contenant des cellules.

12. Mélange de cellules comprenant des cellules tumorales disséminées, **caractérisé en ce qu'**il contient au moins 50 % de cellules tumorales polyclonales, accessible par un procédé conforme à l'une des revendications 1 à 8.

13. Mélange de cellules conforme à la revendication 12, **caractérisé en ce qu'**il contient au moins 90 % de cellules tumorales polyclonales.

14. Mélange de cellules conforme à la revendication 12 ou 13, **caractérisé en ce que** les cellules tumorales sont exemptes de ligands utilisés d'habitude en vue de l'isolement.

15. Procédé d'isolement de constituants cellulaires de cellules tumorales disséminées, dans lequel on récupère des constituants cellulaires, d'une façon connue, à partir d'au moins un mélange de cellules conforme à l'une des revendications 12 à 14 ou d'une fraction d'un tel mélange.

16. Procédé d'établissement de lignées de cellules tumorales disséminées, dans lequel on cultive au moins un mélange de cellules conforme à l'une des revendications 12 à 14 ou une fraction d'un tel mélange.

17. Mélange de cellules conforme à l'une des revendications 12 à 14, conçu pour servir d'agent thérapeutique.

18. Agent pharmaceutique ou vétérinaire, comprenant au moins un mélange de cellules conforme à l'une des revendications 12 à 14 et d'autres auxiliaires de formulation, substances actives et/ou composants d'un test diagnostique.
